# EUROPEAN PATENT SPECIFICATION

(11) **EP 1 955 643 B1**
(45) Date of publication and mention of the grant of the patent: **09.01.2019**
(21) Application number: 06833632.0
(22) Date of filing: 29.11.2006
(51) Int. Cl.: A61B 1/00

(54) **GUIDING LONG MEDICAL MEMBER AND LONG MEDICAL DEVICE**
LANGES MEDIZINISCHES FÜHRUNGSELEMENT UND LANGE MEDIZINISCHE VORRICHTUNG
LONG MEMBRE DE GUIDAGE MÉDICAL ET LONG APPAREIL MÉDICAL

(30) Priority: 01.12.2005 US 741283 P
(43) Date of publication of application: 13.08.2008
(73) Proprietor: Olympus Corporation, Tokyo 192-8507 (JP)
(72) Inventor: MIYAMOTO, Satoshi, Hachioji-shi, Tokyo 192-8507 (JP); KURA, Yasuhito, Hachioji-shi, Tokyo 192-8507 (JP); ONUKI, Yoshio, Hachioji-shi, Tokyo 192-8507 (JP)
(74) Representative: von Hellfeld, Axel
(86) International application number: PCT/JP2006/323828
(87) International publication number: WO 2007/063904

(56) References cited:
- JP-A- 2005 095 590
- JP-A- 2005 118 361
- JP-A- 2005 516 693
- JP-U- 03 036 602
- JP-U- 06 066 609
- US-A- 5 035 231
- US-A- 5 860 913

## Description

### Technical Field

The present invention relates to an elongated medical member with which it becomes easier to make an approach to a bile duct or a pancreatic duct in a body cavity, in particular, from a duodenum via a duodenal papilla.

### Background Art

Up to now, in order to specify a position or a size of a gallstone, a pancreatic stone, or an abnormal area on a duct wall, a close examination with use of an endoscope is conducted. Such an endoscopic examination includes an endoscopic retrograde pancreatico-cholangiography, etc.

According to the angiography, after an angiographic tube is inserted into a duodenal papilla which is an entrance and an exit of a bile duct and a pancreatic duct while a lateral vision endoscope is used, a contrast agent is injected into the bile duct or the pancreatic duct that is a subject of the examination, and X-ray photography is performed. In this way, an operator can specify the position and the size of the gallstone, the pancreatic stone, or the abnormal area on the duct wall on the basis of a bile duct image or a pancreatic image obtained from the X-ray photography.

Then, for the elimination of the bile stone or the pancreatic stone or the treatment of the abnormal area on the duct wall, various treatment instruments are used. It is difficult to introduce these treatment instruments into the bile duct or the pancreatic duct from the duodenal papilla which has a small diameter. For this reason, while using an endoscope, a papillary area is cut with a high frequency knife such as a papillotomy knife, which is one of the treatment instruments, to conduct endoscopic sphincterotomy for increasing the diameter. After that, with use of various treatment instruments such as basket grasping forceps and biopsy forceps, comminution or elimination of the gallstone or the pancreatic stone, treatment of the abnormal area on the duct wall, or the like is performed.

For such a treatment, an elongated catheter which is an auxiliary treatment instrument for inserting various treatment instruments into the bile duct or the pancreatic duct is also used together with an endoscope in some cases. Not being limited to the above-mentioned endoscopic retrograde pancreatico-cholangiography, a catheter used for a medical treatment is disclosed, for example, in US Patent 6,659,981B2. It should be noted that US Patent 6,659,981B2 discloses a technology for the catheter for conveying an internal medical treatment instrument into the heart inside the body.

However, the bile duct or the pancreatic duct accommodates the bile fluid or the pancreatic fluid which is a body fluid, and in the endoscopic retrograde pancreatico-cholangiography using the conventional catheter, there is a problem that after the body fluid is removed at once, it is difficult to inject the contrast agent into the bile duct or the pancreatic duct.

In addition, there is also a problem that when the treatment instrument introduced into the bile duct or the pancreatic duct is taken out and put in within a channel of the conventional catheter, a fluid in the channel is mixed into the bile duct or the pancreatic duct and a shadow appears on an X-ray image, making it difficult to identify an abnormal part. Furthermore, there is also a problem that when the treatment instrument is taken out and put in within the channel of the conventional catheter, the contrast agent injected into the bile duct or the pancreatic duct is sucked into the channel.

Incidentally, the papilla which is an introduction port for the bile duct or the pancreatic duct described above is located at a body cavity wall of the duodenum. For that reason, for an endoscope, in order to include the papilla in a view field, a lateral vision type endoscope provided with the view field in a direction perpendicular to an inserting direction is used. The lateral vision type endoscope includes, for example, as disclosed in Japanese Unexamined Patent Application Publication No. 5-107484, a straight vision/lateral vision switching type endoscope which can freely switch a straight vision type of the view field parallel with the inserting direction and the above-mentioned lateral vision type.

In the conventional straight vision/lateral vision switching type endoscope, during the insertion into the body cavity, a function of only one of the straight vision type and the lateral vision type can be used. For that reason, in a case of the use for the endoscopic retrograde pancreatico-cholangiography, the switching cannot be performed inside the body cavity from the straight vision type effective during the insertion up to the vicinity of the papilla of the duodenum which is a targeted part to the lateral vision type effective when the medical treatment for the bile duct or the pancreatic duct is carried out.

US 5,860,913, which document is considered to represent the most relevant prior art, describes an endoscope having a cover indicator on a distal cover that is aligned with the scope indicator on a main distal part, the distal cover being assembled with the main distal part in an axial direction at a certain insertion angle, and then thereafter being rotated and stopped by a locking part or a hook of the main distal part in order to lock the distal cover onto the main distal part. The main distal part comprises a lumen having an opening proximal of the end of a main distal part, and the distal cover has an opening through which the instrument may be penetrated in order to exit the endoscope to reach the outside.

US 5,035,231 recites an endoscope having an insertion part with an observing window and an illuminating window in a distal tip, and additionally having a probe which may extend from an opening of the insertion portion and may be extended or retracted within the opening to extend beyond or to retract distally from the distal end of the endoscope. The insertion portion further has a grove in a form of a semicircle in which the probe may be extended and retracted. JP 2005-118361 A discloses a double lumen catheter comprising a lumen for transporting blood and lumen for removing blood. The catheter is divided in two halves wherein one half for transporting the blood extends over the other half for removing the blood. At the tip of the blood removing half a protector is formed and the blood removing half comprises a blood removing port on both sides of the back of the protector.

JP2005-095590 A discloses a endoscope for treatment wherein a bending portion is arranged on a flexible tube bending portion comprising inserting channels. One insertion channel projects out of the flexible tube bending portion and an other insertion channel projects out of a end portion of the bending portion of the endoscope.

Thus, the present invention has been made in view of the above-mentioned circumstances, and an object thereof is to realize an elongated medical member with which it is possible to easily insert various treatment instruments and carry out an appropriate medical treatment of an inside of the bile duct or the pancreatic duct without a limitation on the endoscopic retrograde pancreatico-cholangiography.

### Disclosure of Invention

### Means for Solving the Problem

These problems are solved by an elongated medical member according to claim 1.

### Brief Description of the Drawings

FIG. 1 is an entire configuration diagram of an endoscopic system configured to include a lateral vision endoscope and a guide catheter to which an image catheter is inserted according to a first embodiment useful for understanding but not part of the invention.
FIG. 2 shows the guide catheter according to the first embodiment.
FIG. 3 shows a distal end part of the guide catheter according to the first embodiment.
FIG. 4 is a cross sectional diagram of the guide catheter to which the image catheter is inserted according to the first embodiment.
FIG. 5 is a flowchart showing an example of a gallstone elimination surgery based on an endoscopic retrograde pancreatico-cholangiography using the endoscopic system according to the first embodiment.
FIG. 6 shows a state in which the guide catheter is inserted from a duodenal papilla portion into a bile duct according to the first embodiment.
FIG. 7 shows a state in which a contrast agent is injected into the bile duct according to the first embodiment.
FIG. 8 shows a state in which a papillotomy knife is inserted from the duodenal papilla portion into the bile duct according to the first embodiment.
FIG. 9 is an explanatory diagram for an operation of cutting the duodenal papilla portion with the papillotomy knife according to the first embodiment.
FIG. 10 shows a state in which the guide catheter is further inserted into the bile duct according to the first embodiment.
FIG. 11 shows a state in which basket grasping forceps are inserted into the bile duct according to the first embodiment.
FIG. 12 shows a state in which a gallstone in the bile duct is grasped by the basket grasping forceps according to the first embodiment.
FIG. 13 shows a state in which the basket grasping forceps grasping the gallstone are pulled into a duodenum according to the first embodiment.
FIG. 14 illustrates a state in which a guide portion of the guide catheter is inserted into a pancreatic duct according to the first embodiment.
FIG. 15 illustrates a state in which the guide portion of the guide catheter is further inserted into a pancreatic duct from the state in FIG. 14 and the image catheter is inserted into the bile duct according to the first embodiment.
FIG. 16 is an explanatory diagram for an operation of inserting the guide portion of the guide catheter into the bile duct and cutting the duodenal papilla portion with a needle-shaped knife according to the first embodiment.
FIG. 17 illustrates a state in which the guide catheter is inserted into the bile duct to suck the bile fluid, and also a contrast agent is injected into the bile duct according to the first embodiment.
FIG. 18 illustrates a state in which a gallstone in the bile duct is captured by basket grasping forceps while observing the inside of the bile duct with the image catheter protruding from the guide portion according to the first embodiment.
FIG. 19 is an entire configuration diagram of an endoscope according to a second embodiment useful for understanding but not part of the invention.
FIG. 20 is a plan view of a distal end surface of the endoscope of FIG. 19 according to the second embodiment.
FIG. 21 is a cross sectional view of a distal end portion of the endoscope in the state shown in FIG. 20 according to the second embodiment.
FIG. 22 is a cross sectional view of the distal end portion of the endoscope in a state in which a view direction of an image catheter is changed according to the second embodiment.
FIG. 23 is a plan view of the distal end surface of the endoscope in the state shown in FIG. 22 according to the second embodiment.
FIG. 24 shows a state in which the endoscope is inserted in the body cavity according to the second embodiment.
FIG. 25 shows a state in which the endoscope reaches a point in the vicinity of the duodenal papilla portion according to the second embodiment.
FIG. 26 is a cross sectional view of a distal end portion of the endoscope of a first modification example according to the second embodiment.
FIG. 27 is a cross sectional view of the distal end portion of the endoscope in a state in which a view direction of an image catheter of the first modification example is changed according to the second embodiment.
FIG. 28 is a cross sectional view of a distal end portion of the endoscope of a second modification example according to the second embodiment.
FIG. 29 is a cross sectional view of the distal end portion of the endoscope in a state in which a view direction of an image catheter of the second modification example is changed according to the second embodiment.
FIG. 30 is an entire configuration diagram of an endoscopic system configured to include a guide catheter according to the second embodiment.
FIG. 31 shows a configuration of a distal end part of the guide catheter according to a third embodiment useful for understanding but not part of the invention.
FIG. 32 is a cross sectional view of the distal end part of the guide catheter according to the third embodiment.
FIG. 33 is a side view of the distal end part of the guide catheter in a state in which the image catheter provided with a bending portion where the distal end part is held by a holding portion is penetrated according to the third embodiment.
FIG. 34 is a side view of the distal end part of the guide catheter in a state in which the image catheter is penetrated, representing a state in which the image catheter is bent according to the third embodiment.
FIG. 35 shows a configuration of a distal end part of the guide catheter according to a fourth embodiment useful for understanding but not part of the invention.
FIG. 36 is a cross sectional view of the distal end part of the guide catheter according to the fourth embodiment.
FIG. 37 is a cross sectional view of the distal end part of the guide catheter which is a modification example according to the fourth embodiment.
FIG. 38 is an explanatory diagram for an action of the guide catheter of FIG. 37 which is the modification example according to the fourth embodiment.
FIG. 39 is a perspective view of the distal end part of the guide catheter according to a fifth embodiment useful for understanding but not part of the invention.
FIG. 40 is a perspective view of the distal end part of the guide catheter, representing a first modification example according to the fifth embodiment.
FIG. 41 is a perspective view of the distal end part of the guide catheter representing a second modification example according to the fifth embodiment.
FIG. 42 illustrates a use example of the guide catheter of FIG. 39 according to the fifth embodiment.
FIG. 43 illustrates a use example of the guide catheter of FIG. 39 according to the fifth embodiment.
FIG. 44 shows a configuration of a distal end part of the guide catheter according to a sixth embodiment useful for understanding but not part of the invention.
FIG. 45 shows a configuration of a distal end part of the guide catheter according to a seventh embodiment useful for understanding but not part of the invention.
FIG. 46 shows the distal end part of the guide catheter according to the seventh embodiment in which a distal end part of the papillotomy knife inserted to this guide catheter is shaped arcuate according to the seventh embodiment.
FIG. 47 is an explanatory diagram for an operation of cutting the duodenal papilla with the papillotomy knife according to the seventh embodiment.
FIG. 48 shows a state in which basket grasping forceps grasping a gallstone are pulled to the duodenum according to the seventh embodiment.
FIG. 49 is a cross sectional view of an endoscope channel and a guide catheter penetrated through this endoscope channel according to an eighth embodiment useful for understanding but not part of the invention.
FIG. 50 is a cross sectional view of the guide catheter and an image catheter penetrated through this guide catheter according to the eighth embodiment.
FIG. 51 illustrates indices for respectively regulating an inserting direction about a long axis on a channel opening portion on an operation portion side of the lateral vision endoscope, the distal part of the guide catheter, a treatment instrument penetration portion of the guide catheter, and the distal part of the image catheter which is a treatment instrument according to the eighth embodiment.
FIG. 52 illustrates a rail-shaped groove portion formed from the guide portion of the guide catheter to the proximal end of the guide catheter insertion portion according to the eighth embodiment.
FIG. 53 is a cross sectional view of the guide catheter of FIG. 52 in a state in which a treatment instrument is penetrated according to the eighth embodiment.
FIG. 54 shows a configuration of a distal end part of the guide catheter according to a ninth embodiment useful for understanding but not part of the invention.
FIG. 55 is a view of the guide catheter as seen from the distal end according to the ninth embodiment.
FIG. 56 is a cross sectional view of the distal end part of the guide catheter which is a first modification example according to the ninth embodiment.
FIG. 57 illustrates a configuration of the distal end part of the guide catheter which is a second modification example according to the ninth embodiment.
FIG. 58 illustrates a state in which the guide catheter is inserted into the bile duct from the duodenal papilla portion which is the second modification example according to the ninth embodiment.
FIG. 59 is a cross sectional view of a distal end part of a guide catheter to which basket grasping forceps are inserted according to a tenth embodiment useful for understanding but not part of the invention.
FIG. 60 shows a state in which a guide portion of the guide catheter to which the basket grasping forceps are inserted is inserted to the bile duct according to the tenth embodiment.
FIG. 61 is a plan view showing one side of a distal end portion of an endoscope according to an eleventh embodiment useful for understanding but not part of the invention.
FIG. 62 is a perspective view showing the distal end portion of the endoscope according to the eleventh embodiment.
FIG. 63 is a plan view showing one side of a distal end portion of an endoscope according to a twelfth embodiment useful for understanding but not part of the invention.
FIG. 64 is a plan view showing a distal end surface of the distal end portion of the endoscope according to the twelfth embodiment.
FIG. 65 is a cross sectional view of the distal end portion of the endoscope according to the twelfth embodiment.
FIG. 66 shows a hole section inside the distal end portion, a straight vision hole section, or a lateral vision hole section, being a partial cross sectional view of the distal end portion which shows a scope penetration concave section according to the twelfth embodiment.
FIG. 67 is a cross sectional view of the distal end portion of the endoscope in a case where the guide catheter provided with a holding portion to a guide portion and the image catheter are used according to the twelfth embodiment.
FIG. 68 illustrates a state in which the distal end portion of the endoscope based on a configuration corresponding to FIG. 67 is inserted to a location in the vicinity of the papilla portion and the image catheter held by the holding portion of the guide portion slightly protrudes according to the twelfth embodiment.
FIG. 69 illustrates a state in which the guide portion is inserted into the bile duct together with the image catheter according to the twelfth embodiment.
FIG. 70 illustrates a state in which a distal end portion of a guide catheter insertion portion of the guide catheter is inserted into the bile duct and the image catheter is removed from the holding portion to pick up an image of the papilla portion according to the twelfth embodiment.
FIG. 71 is an explanatory diagram for explaining a configuration in which the distal end portion of the endoscope is provided with a holding portion, representing a modification example according to the twelfth embodiment.

Hereinafter, embodiments of the present invention will be described with reference to the drawings.

### (First Embodiment)

First of all, a description will be given of a first embodiment useful for understanding but not part of the invention.

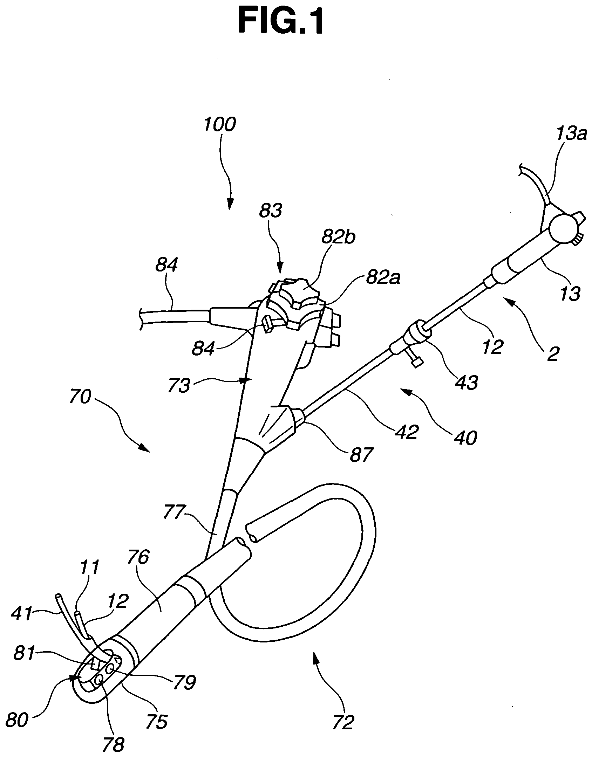

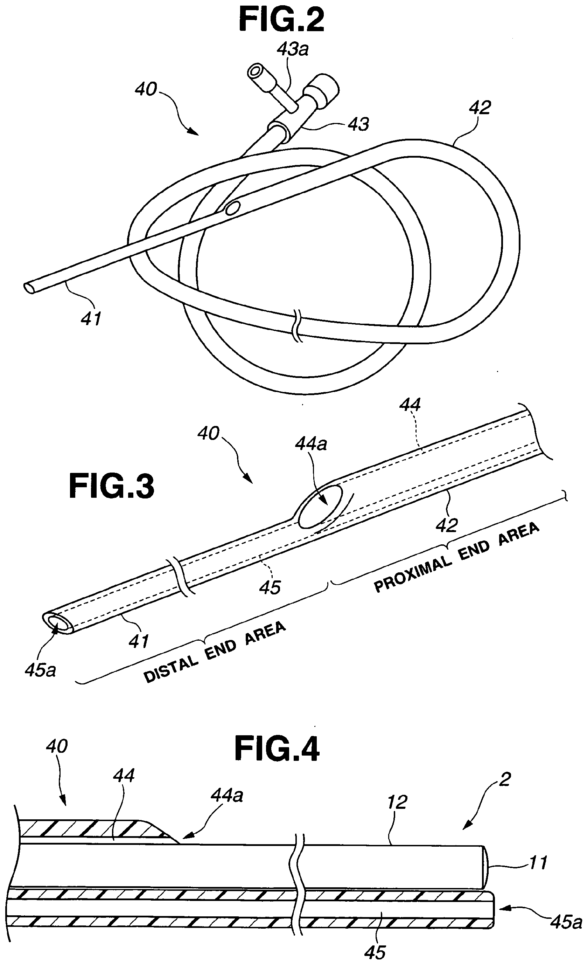

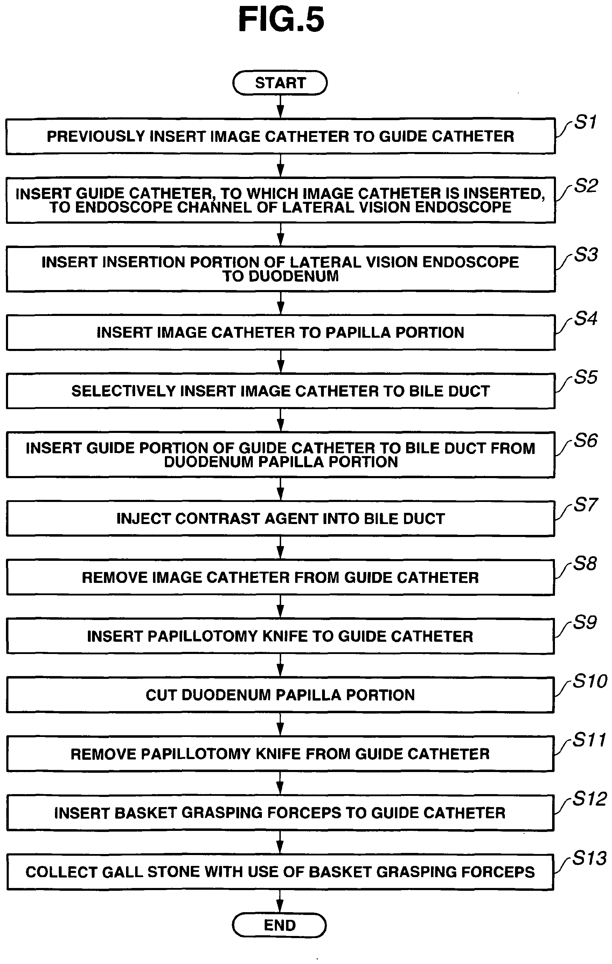

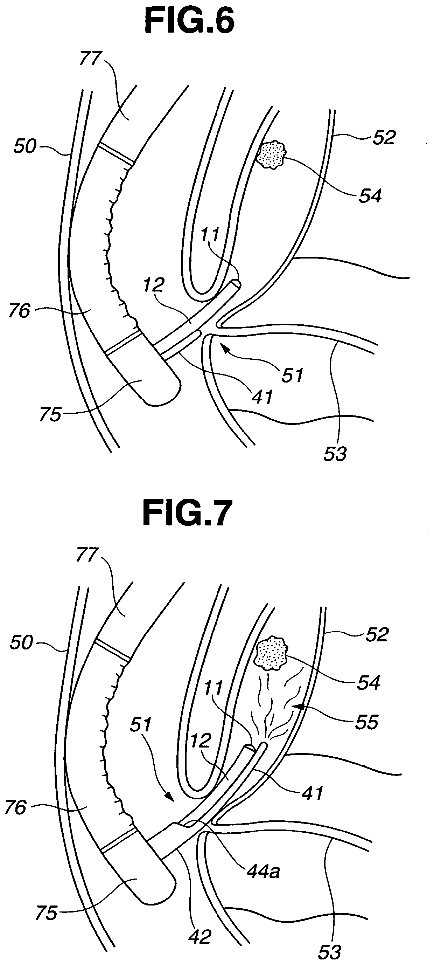

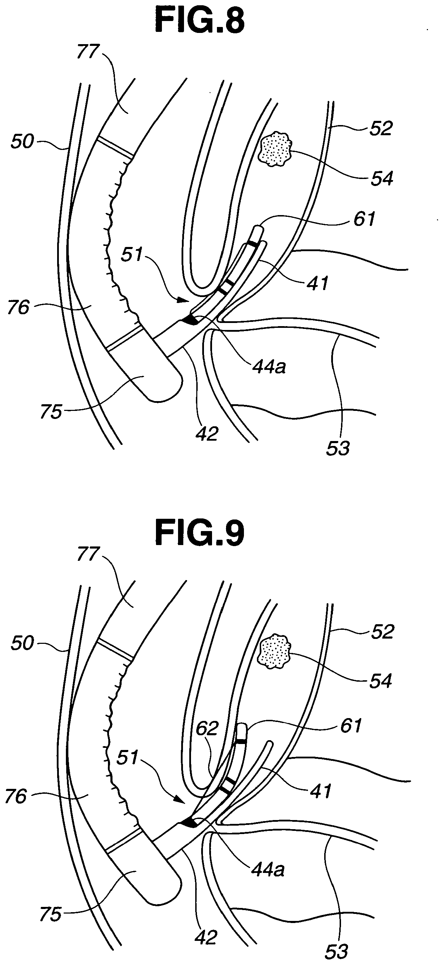

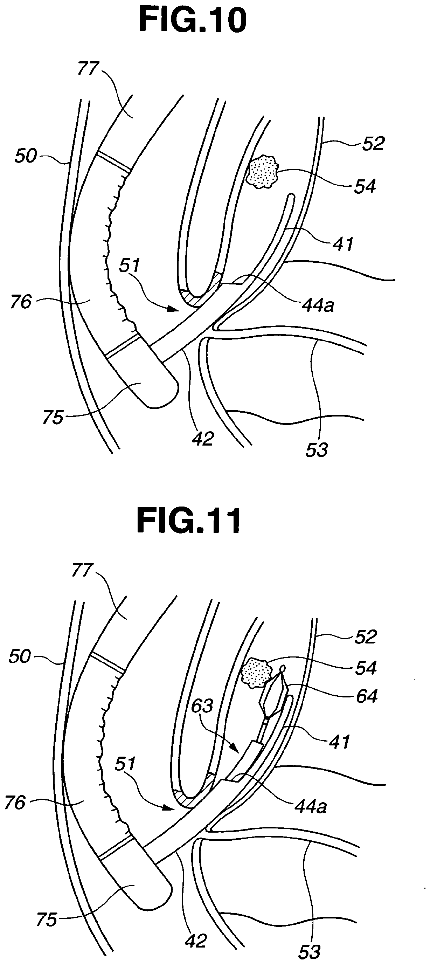

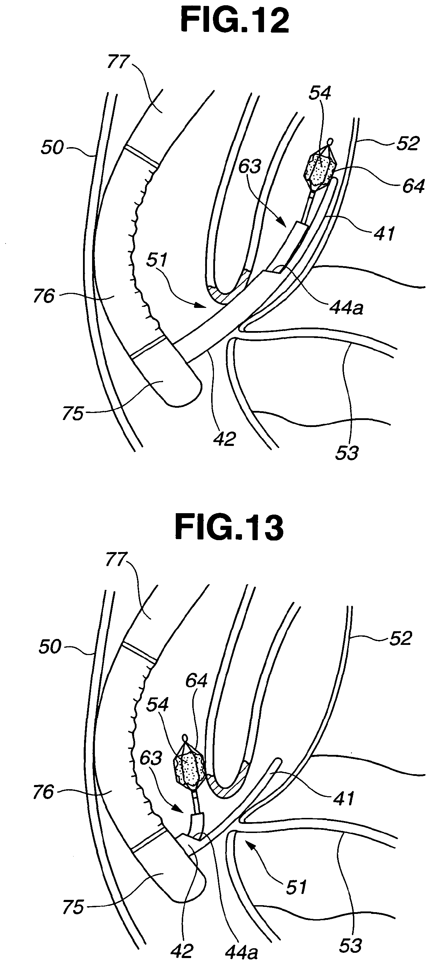

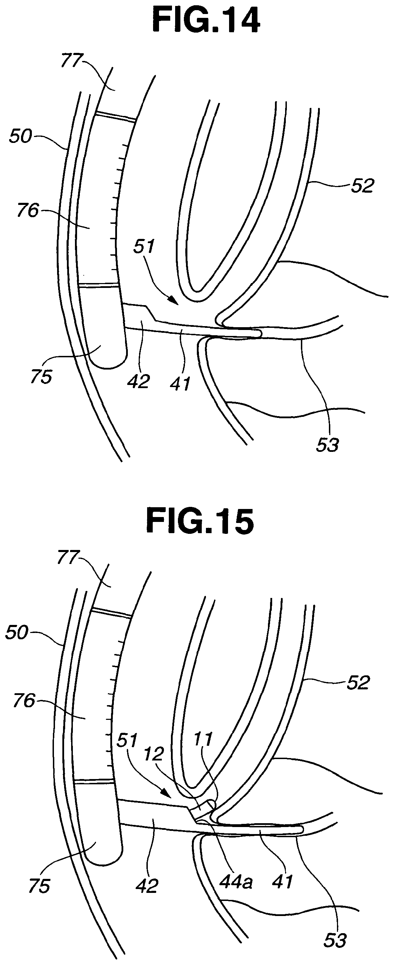

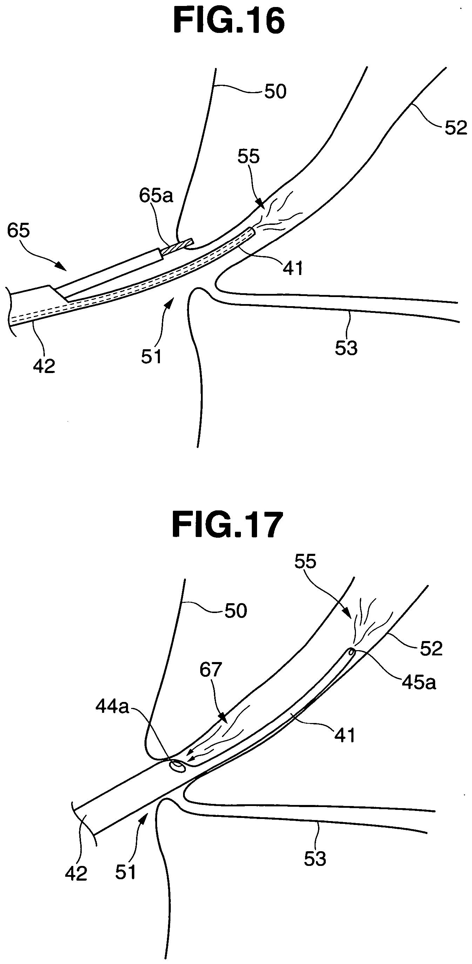

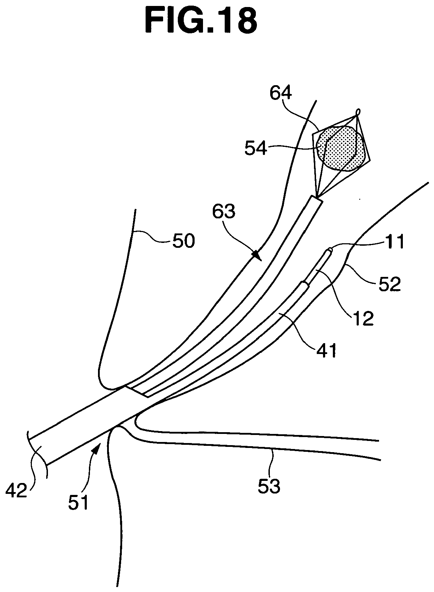

FIGS. 1 to 18 represent the first embodiment: FIG. 1 is an entire configuration diagram of an endoscopic system configured to include a lateral vision endoscope and a guide catheter to which an image catheter is inserted, FIG. 2 shows the guide catheter, FIG. 3 shows a distal end part of the guide catheter, FIG. 4 is a cross sectional diagram of the guide catheter to which the image catheter is inserted, FIG. 5 is a flowchart showing an example of a gallstone elimination surgery based on an endoscopic retrograde pancreatico-cholangiography using the endoscopic system, FIG. 6 shows a state in which the guide catheter is inserted from a duodenal papilla portion into a bile duct, FIG. 7 shows a state in which a contrast agent is injected into the bile duct, FIG. 8 shows a state in which a papillotomy knife is inserted from the duodenal papilla portion into the bile duct, FIG. 9 is an explanatory diagram for an operation of cutting the duodenal papilla portion with a papillotomy knife, FIG. 10 shows a state in which the guide catheter is further inserted into the bile duct, FIG. 11 shows a state in which basket grasping forceps are inserted into the bile duct, FIG. 12 shows a state in which a gallstone in the bile duct is grasped by the basket grasping forceps, and FIG. 13 shows a state in which the basket grasping forceps grasping the gallstone are pulled into a duodenum, FIG. 14 illustrates a state in which a guide portion of the guide catheter is inserted into a pancreatic duct, FIG. 15 illustrates a state in which the guide portion of the guide catheter is further inserted into a pancreatic duct from FIG. 14 and the image catheter is inserted into the bile duct, FIG. 16 is an explanatory diagram for an operation of inserting the guide portion of the guide catheter into the bile duct and cutting the duodenal papilla portion with a needle-shaped knife, FIG. 17 illustrates a state in which the guide catheter is inserted into the bile duct to suck the bile fluid, and also a contrast agent is injected into the bile duct, and FIG. 18 illustrates a state in which a gallstone in the bile duct is captured by basket grasping forceps while observing the inside of the bile duct with the image catheter protruding from the guide portion.

First, a lateral vision endoscope 70 functioning as an elongated medical member, which is shown in FIG. 1, will be described briefly. The lateral vision endoscope 70 is configured to mainly include an elongated insertion portion 72 having a flexibility to be inserted into a body cavity, an operation portion 73 provided adjacent to a proximal end portion of the insertion portion 72, and a universal code 74 extending from a side portion of the operation portion 73 and having at its proximal end portion a connector portion detachably attached to a connection portion such as a light source device or a video processor, etc., which is not shown in the drawing.

In the insertion portion 72, in the order from a distal end side, there are arranged adjacent to each other a distal end portion 75 that is formed of a rigid member, a bending portion 76 that is formed to be bendable in up, down, left, and right directions, for example, by connecting a plurality of bending pieces to each other so as to freely turn, and a flexible tube portion 77 having a flexibility.

On a side portion of the distal end portion 75, there are arranged a lens attachment hole portion 78 in which an illumination window for emitting illumination light functions as one end surface, an observation window 79 for picking up an optical image, and a storage room 80. In the storage room 80, a treatment instrument raising table 81 is provided for changing in a desired direction a protruding direction of a distal end portion of a guide catheter 40 through which an image catheter 2 functioning as an optical device to be described below is penetrated.

On a side surface of the operation portion 73, bending operation knobs 82a and 82b are arranged so as to be overlapped, for changing a bending direction of a bending portion 6 through operation at hands. An optical image picked up by image pickup means such as a CCD or a CMOS, which is not shown in the drawing, incident from the observation window 79 is displayed on a monitor not shown via a video processor, not shown, that is electrically connected to a proximal end of a universal code 4. In addition, the operation portion 73 includes a guide element raiser lever 84 for changing the protruding direction of the treatment instrument while a treatment instrument raising table 81 is turned, a water supply button for controlling a water supply function, a suction button for controlling a suction function, an operation portion side channel opening portion 87 functioning as an entrance of a treatment instrument penetration channel (hereinafter, referred to as an endoscope channel) whose one end portion is in communication with a storage room 10, and the like. In other words, the storage room 80 doubles as an exit of the treatment instrument penetration channel. Furthermore, the operation portion 73 includes various switches 83 for remotely performing light amount adjustment of the light source device that is not shown in the drawing.

The image catheter 2 functioning as an image pickup apparatus includes a distal end surface 11 having an optical system, an elongated scope insertion portion 12 which is an introduction portion, an operation portion 13 provided to be connected to a proximal end of the scope insertion portion 12, having an electric code extended to be connected to a video processor that is not shown in the drawing, and also accommodates therein an image pickup element unit such as a CCD or a CMOS, not shown, configuring an image pickup portion for picking up an image pickup light incident from the distal end surface 11 and transferred by an observation light transfer member which is not shown. It should be noted that the image catheter 2 may also employ a fiber scope type configuring the observation light transfer member with which visual recognition can be achieved with an eye piece portion that does not have the above-mentioned image pickup means.

The guide catheter 40 functioning as a guiding elongated medical member is inserted from the operation portion side channel opening portion 87 at the operation portion 73 of the lateral vision endoscope 70 into the insertion portion 72, and its distal end part is guided from the distal end portion 75. The guide catheter 40 is an elongated tube body formed of a synthetic resin, a rubber, or the like, which has a flexibility and a biocompatibility. Through the guide catheter 40 to be inserted into the insertion portion 72 of the lateral vision endoscope 70, further, the image catheter 2 or various treatment instruments can be freely penetrated.

As shown in FIG. 2, the guide catheter 40 includes a small diameter guide portion 41 which is a distal end area with a small diameter having a predetermined length at the distal end part, a guide catheter insertion portion 42 which is a proximal end area having the guide portion 41 extended from a periphery of the distal end surface, and a scope/treatment instrument penetration portion 43 provided adjacent to the proximal end of the guide catheter insertion portion 42.

The scope/treatment instrument penetration portion 43 is of a substantially tube shape, and has an opening part at a proximal end surface for inserting the image catheter 2 or various treatment instruments into the guide catheter insertion portion 42 and a fluid supply portion 43a extended from a side peripheral surface to be connected to a syringe or the like for supplying a fluid.

Also, as shown in FIGS. 3 and 4, the guide catheter 40 is formed of a double rumen tube having an outer diameter of, for example, 6 mm or smaller, which includes a fluid supply channel 45 arranged from an opening portion 45a formed at the distal end surface of the guide portion 41 to the fluid supply portion 43a of the scope/treatment instrument penetration portion 43, and a scope/treatment instrument channel 44 arranged from an opening portion 44a formed at the distal end surface of the guide catheter insertion portion 42 to the scope/treatment instrument penetration portion 43. That is, the scope/treatment instrument channel 44 configuring a first lumen arranged to the guide catheter insertion portion 42 becomes a near end which is a root portion of the guide portion 41 where the distal end surface extends, and is opened at the opening portion 44a of the near end and also opened at a remote end of the guide portion 41 which is a proximal end.

Therefore, in the guide catheter 40, the image catheter 2 also functioning as an operation medical member or various treatment instruments are inserted from the scope/treatment instrument penetration portion 43 into the scope/treatment instrument channel 44. Then, the image catheter 2 or various treatment instruments are guided out from the opening portion 44a at the distal end surface of the guide catheter insertion portion 42.

It should be noted that the outer diameter of the image catheter 2 according to the present embodiment is, for example, about 2 mm or smaller. For that reason, the scope/treatment instrument channel 44 has a channel diameter set so as to enable the penetration of the image catheter 2. In addition, the outer diameters of various treatment instruments are about 10 Fr. For that reason, the scope/treatment instrument channel 44 has a channel diameter set so as to enable the penetration of various treatment instruments.

Also, according to the present embodiment, as will be described later, the guide portion 41 of the guide catheter 40 has an outer diameter determined so as to pass through a papilla portion 51 of the duodenum 50.

As shown in FIG. 1, among the lateral vision endoscope 70, the guide catheter 40, the image catheter 2, and various treatment instruments, a configuration at least including two of the lateral vision endoscope 70, the guide catheter 40, and the image catheter 2 configures the endoscopic system 100 functioning as the elongated medical apparatus according to the present embodiment.

As in the above-mentioned manner, while using the endoscopic system 100 according to the present embodiment configured to include the lateral vision endoscope 70, the guide catheter 40, and the image catheter 2, through the endoscopic retrograde pancreatico-cholangiography, for example, in a bile passage/pancreatic duct area in which the papilla portion of the duodenum becomes an entrance, an example of the endoscopic papilla sphincterotomy for discharging the gallstone in the bile duct 52 will be described mainly with reference to FIG. 1 and FIGS. 6 to 13 according to the first embodiment. In addition, hereinafter, according to a flow example of each step (S) shown in a flowchart of FIG. 5, an operation for taking out the gallstone in the bile duct 52 will be described.

First, the operator previously inserts the scope insertion portion 12 of the image catheter 2 from the scope/treatment instrument penetration portion 43 to the scope/treatment instrument channel 44 of the guide catheter 40 (S1). Next, the operator inserts the guide catheter 40 to which the image catheter 2 is inserted, from the operation portion side channel opening portion 87 of the lateral vision endoscope 70 to the endoscope channel (S2).

Then, while the operator visually checks the inside of the body cavity on the basis of an endoscopic image picked up by the lateral vision endoscope 70, the distal end portion 75 of the insertion portion 72 of the lateral vision endoscope 70 is inserted to the duodenum 50 (S3). At this time, the operator appropriately performs a bending operation on the bending portion 6 in accordance with the bending state of the body cavity or performs a twist operation on the insertion portion 72. In addition, when the operator inserts the distal end portion 75 of the insertion portion 72 to the vicinity of the papilla portion 51, on the basis of the endoscopic image of the lateral vision endoscope 70, a position of the papilla portion 51 is identified.

Next, as shown in FIG. 6, the distal end part of the image catheter 2 is inserted from the papilla portion 51 into the bile duct 52 which is bile passage/pancreatic duct area (S4). At this time, the operator can easily insert the image catheter 2 to the papilla portion 51 on the basis of the endoscopic image of the lateral vision endoscope 70 and the image of the image catheter 2.

Next, while visually checking the image of the image catheter 2, the operator inserts the image catheter 2 to the bile duct 52 selectively (S5). In other words, herein, for the operation for taking out the gallstone 54 in the bile duct 52, the operator selects bile duct 52, but when a pancreatic duct 53 is inspected and treated, by visually checking the image of the image catheter 2, the image catheter 2 can be easily inserted to the pancreatic duct 53 as well. That is, on the basis of the image of the image catheter 2, the operator can easily distinguish the bile duct 52 and the pancreatic duct 53 which are located deeper than the papilla portion 51 from each other, and while visually checking, the image catheter 2 can be inserted to the bile duct 52 or the pancreatic duct 53.

Next, the operator inserts, along with the image catheter 2, the guide portion 41 of the guide catheter 40 into the bile duct 52 (S6). At this time, as the operator locates the distal end surface 11 of the image catheter 2 slightly protruding from the opening portion 44a of the distal end surface of the guide catheter insertion portion 42, while the papilla portion 51 is visually checked on the basis of the endoscopic image of the lateral vision endoscope 70, the guide portion 41 can be inserted to the bile duct 52. In addition, with use of transmitted light due to irradiation of illumination light from LEDs 23 at the distal end portion 5, the operator can easily distinguish the bile duct 52 and the pancreatic duct 53 which are located deeper than the papilla portion 51 from each other as the distal end surface of the image catheter 2 is located in the vicinity of the papilla portion 51, and while visually checking, the guide portion 41 can be inserted to the bile duct 52.

Next, the operator injects, as shown in FIG. 7, the contrast agent 55 into the bile duct 52 (S7). At this time, the operator injects, with use of the syringe or the like, the contrast agent 55 from the fluid supply portion 43a at the scope/treatment instrument penetration portion 43 of the guide catheter 40 to the fluid supply channel 45. The contrast agent 55 is supplied via the fluid supply channel 45 to the opening portion 45a of the guide portion 41 into the bile duct 52. In this way, the operator can pickup an image of the bile duct 52 to which the contrast agent 55 is inserted through X lay.

In addition, as the operator can easily insert, as shown in FIG. 7, the image catheter 2 along the guide portion 41 into a further deep portion of the bile duct 52, the injection state of the contrast agent 55, an abnormal area of a duct wall of the bile duct 52, a location and a size of the gallstone 54, and the like can be visually checked.

Next, the operator removes the image catheter 2 from the guide catheter 40 (S8). Then, the operator inserts a papillotomy knife 61, which is one of the treatment instruments, from the scope/treatment instrument penetration portion 43 of the guide catheter 40 into the scope/treatment instrument channel 44 (S9). At this time, while the operator checks the X-ray image, as shown in FIG. 8, a distal end part functioning as a treatment portion of the papillotomy knife 61 which is an operation medical member, protruding from the opening portion 44a of the distal end surface of the guide catheter insertion portion 42 can be inserted along the guide portion 41 from the papilla portion 51 into the bile duct 52.

Then, the operator cuts the papilla portion 51 (S10). To be specific, the operator pulls a wire cutter 62 as shown in FIG. 9, the distal end part of the papillotomy knife 61 is shaped arcuate, and a high frequency is applied to the wire cutter 62, thereby cutting the papilla portion 51. As a result, various treatment instruments can be easily inserted from the papilla portion 51 having a small opening into the bile duct 52.

Next, the operator removes the papillotomy knife 61 from the guide catheter 40 (S11). It should be noted that as the operator cuts the papilla portion 51 as shown in FIG. 10, the distal end part of the guide catheter insertion portion 42 of the guide catheter 40 can be easily inserted into the bile duct 52.

Next, the operator inserts basket grasping forceps 63, which are one of the treatment instruments configuring the operation medical member, from the scope/treatment instrument penetration portion 43 of the guide catheter 40 into the scope/treatment instrument channel 44 (S12). Then, the operator protrudes, while observing the X-ray image, as shown in FIG. 11, a basket wire 64 which is a treatment portion of the basket grasping forceps 63 from the opening portion 44a of the guide catheter insertion portion 42.

Next, the operator uses the basket grasping forceps 63 to collect the gallstone 54 (S13). At this time, the operator puts the basket wire 64 of the basket grasping forceps 63 close to the gallstone 54 and also performs a rotation operation, whereby, as shown in FIG. 12, the gallstone 54 is put in the basket wire 64 for grasping. Next, the operator pulls, together with the basket grasping forceps 63, the guide catheter insertion portion 42 of the guide catheter 40 via the papilla portion 51 from the bile duct 52, as shown in FIG. 13, into the duodenum 50, whereby the gallstone 54 grasped by the basket wire 64 is taken out from the bile duct 52. It should be noted that the papilla portion 51 is cut as in the above-mentioned manner, and the gallstone 54 can easily pass through the papilla portion 51.

As described above, with the endoscopic system 18 according to the present embodiment, the guide portion 41 of the guide catheter 40 can be inserted from the papilla portion 51 into the bile duct 52 or the pancreatic duct 53 which is the bile passage/pancreatic duct area with ease and reliability and along the guide portion 41, the insertability of the image catheter 2, various treatment instruments, and the like into the bile duct 52 or the pancreatic duct 53 can be increased.

It should be noted that as shown in FIG. 14, the operator inserts the guide portion 41 of the guide catheter 40 into the pancreatic duct 53. Then, the operator further advances the guide catheter 40 so that the opening portion 44a at the distal end of the guide catheter insertion portion 42 is located at the papilla portion 51 as shown in FIG. 15 to configure the guide portion 41 inserted into the pancreatic duct 53 as a so-called anchor role, whereby the guide catheter 40 can be set in a stable state. In this state, from the opening portion 44a of the guide catheter 40, the operator can easily insert the treatment instrument, for example, on the paper, the scope insertion portion 12 of the image catheter 2 into the bile duct 52.

In addition, the above-mentioned treatment instrument used for cutting the papilla portion 51 is not limited to the papillotomy knife 61, and as shown in FIG. 16, for example, a treatment instrument such as a needle-shaped knife 65 provided with a knife portion 65a adapted to cut a living body tissue on the basis of a high frequency may be used. In a case of using such a treatment instrument too, the papilla portion 51 which is a treated portion may be cut and treated with use of a video picked up on the basis of a contrast agent 55 injected into the bile duct 52 with the X ray or a video picked up through the endoscope 1.

Furthermore, as shown in FIG. 17, in a state in which the distal end part of the guide catheter insertion portion 42 of the guide catheter 40 is inserted, herein, into the bile duct 52, the contrast agent 55 is injected from the opening portion 45a of the fluid supply channel 45 at the distal end of the guide portion 41 into the bile duct 52, and also a bile fluid 67 which is a body fluid inside the bile duct 52 may be sucked from the opening portion 44a at the distal end of the guide catheter insertion portion 42 via the treatment instrument channel 44. As a result, the contrast agent 55 can be uniformly loaded within the bile duct 52.

It should be noted that in the description in FIG. 17, the case for reliably load the contrast agent 55 within the bile duct 52 has been described, but a fluid supply from an opening portion 45a of the fluid supply channel 45 is performed and this fluid is sucked from the opening portion 44a via the treatment instrument channel 44, whereby cleaning within the bile duct 52 can be also performed.

In addition, the guide catheter 40 is inserted into the bile duct 52, and as shown in FIG. 18, at the time of the treatment using the treatment instrument, herein, the basket grasping forceps 63, the image catheter 2 is inserted into the fluid supply channel 45 to allow the distal end surface 11 of the scope insertion portion 12 to protrude from the opening portion 45a at the distal end of the guide portion 41, whereby an image of the treatment state inside the bile duct 52 may be picked up by the image catheter 2.

### (Second Embodiment)

Next, a second embodiment useful for understanding but not part of the invention will be described. According to the present embodiment, an endoscopic system including a guide catheter used together with the endoscope 1 will be described.

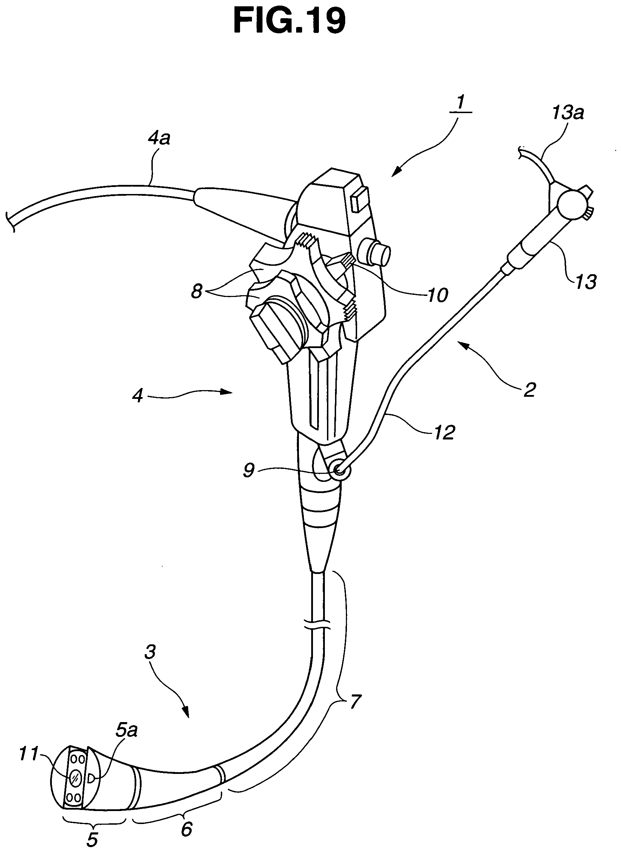

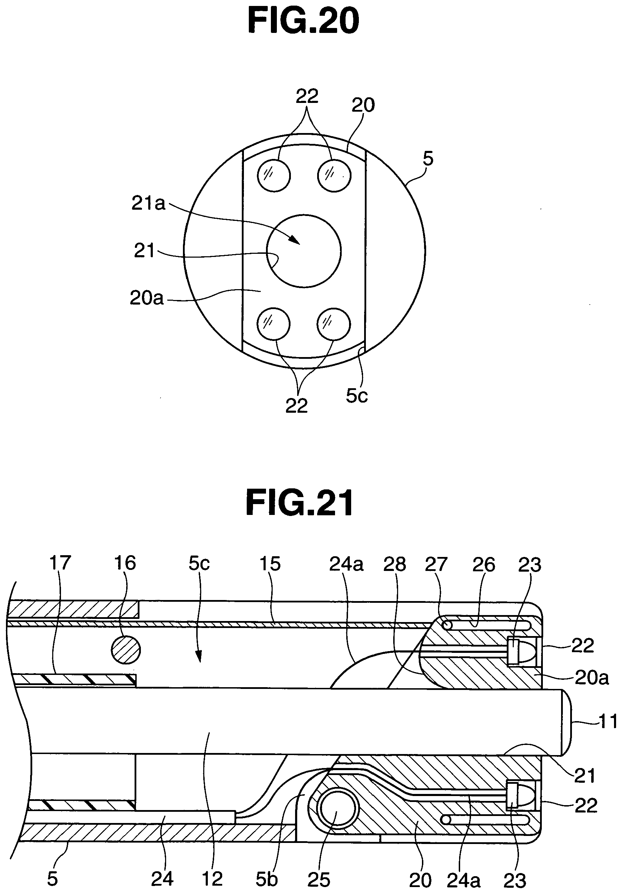

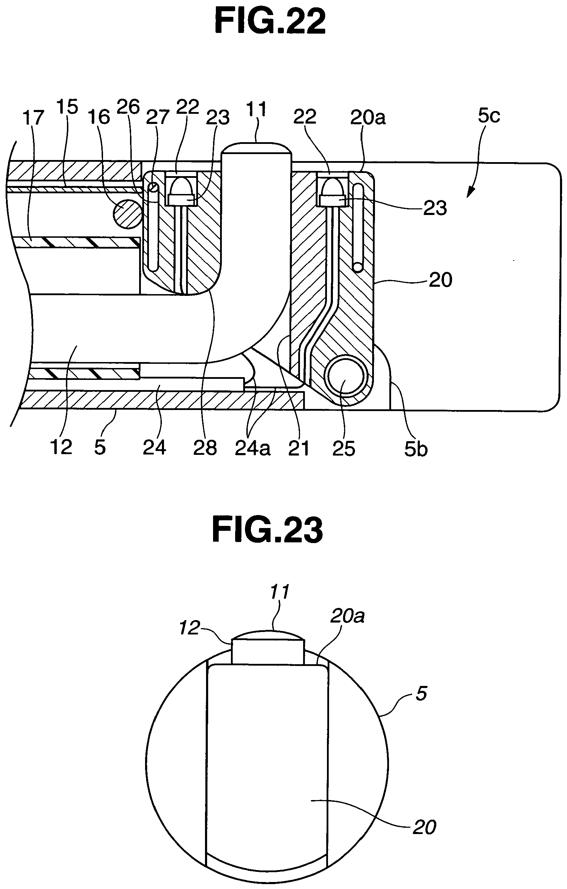

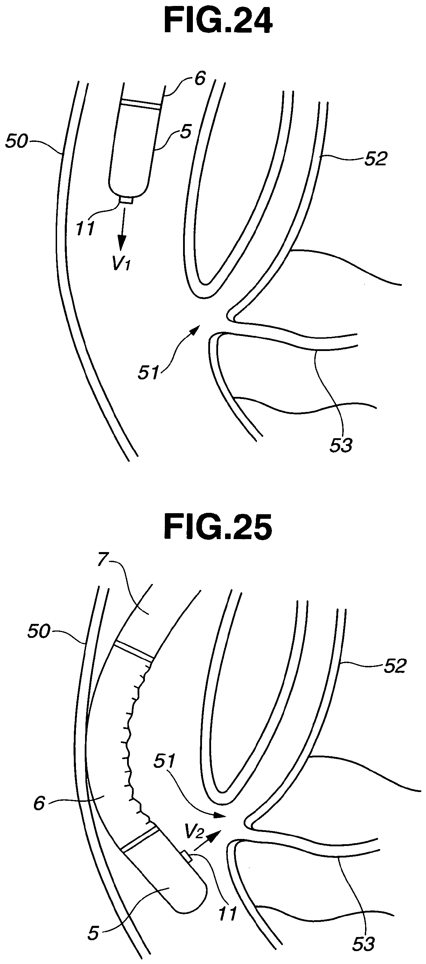

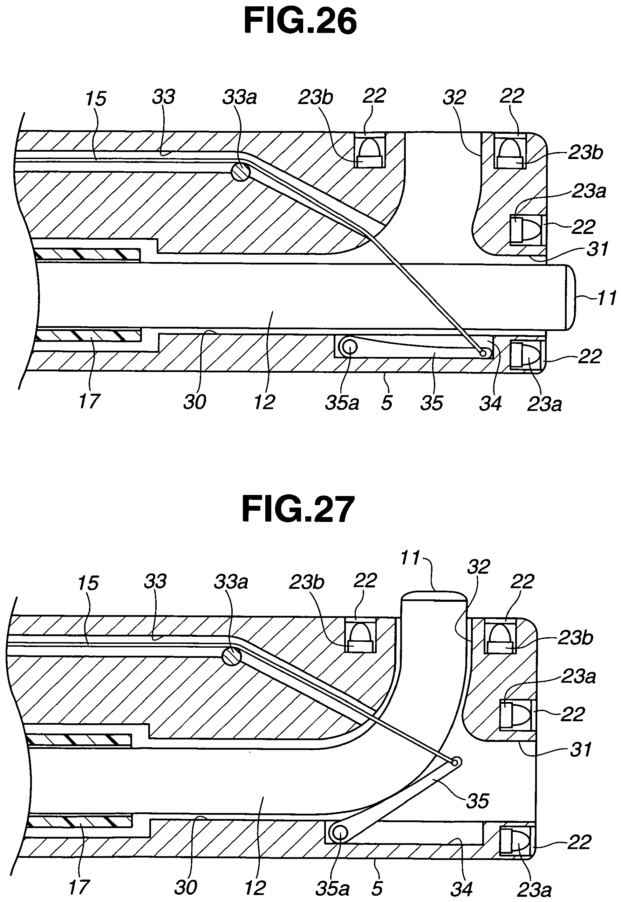

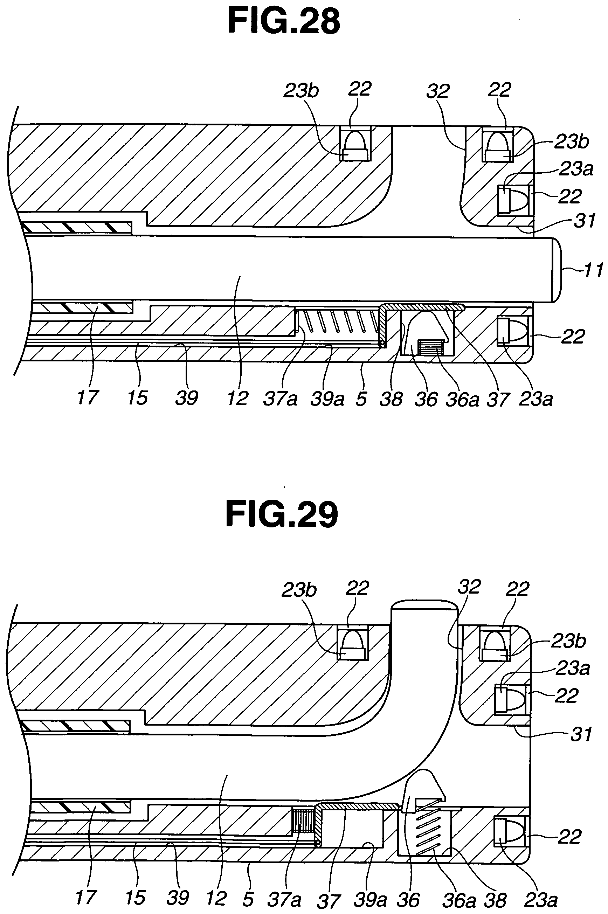

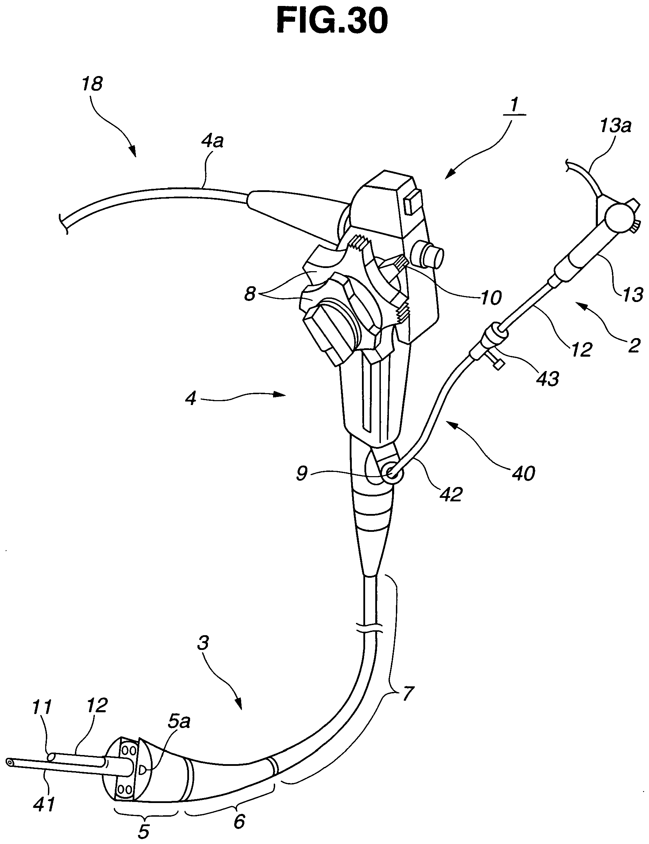

FIGS. 19 to 30 represent the second embodiment of the present invention: FIG. 19 is an entire configuration diagram of an endoscope, FIG. 20 is a plan view of a distal end surface of the endoscope of FIG. 19, FIG. 21 is a cross sectional view of a distal end portion of the endoscope in the state shown in FIG. 20, FIG. 22 is a cross sectional view of the distal end portion of the endoscope in a state in which a view direction of an image catheter is changed, FIG. 23 is a plan view of the distal end surface of the endoscope in the state shown in FIG. 22, FIG. 24 shows a state in which the endoscope is inserted in the body cavity, FIG. 25 shows a state in which the endoscope reaches a point in the vicinity of the duodenal papilla, FIG. 26 is a cross sectional view of a distal end portion of the endoscope according to a first modification example, FIG. 27 is a cross sectional view of the distal end portion of the endoscope in a state in which a view direction of an image catheter of the first modification example is changed, FIG. 28 is a cross sectional view of a distal end portion of the endoscope according to a second modification example, FIG. 29 is a cross sectional view of the distal end portion of the endoscope in a state in which a view direction of the image catheter 2 according to the second modification example is changed, and FIG. 30 is an entire configuration diagram of an endoscopic system configured to include a guide catheter.

It should be noted that in the description on the present embodiment, the same numerals are used for the configurations of the first embodiment described above and the detailed description will be omitted.

As shown in FIG. 19, the endoscope 1 functioning as the elongated medical member according to the present embodiment includes the image catheter 2 functioning as an optical device, an elongated insertion portion 3 to be inserted into a body cavity, and the operation portion 4 for operating various functions.

The insertion portion 3 includes, in the order from its distal end, the distal end portion 5, the bending portion 6, and a flexible portion 7. The bending portion 6 allows free bending operations by bending operation knobs 8 of the operation portion 4 as will be described later. The flexible portion 7 is a flexible tube body.

The operation portion 4 is coupled to the proximal end of the flexible portion 7 and has a universal cable 4a to be connected to a control device doubling as power supply, which is not shown in the drawing, extending from its proximal end part. According to the present embodiment, the operation portion 4 includes, on one side surface, two bending operation knobs 8 for performing bending operations on the bending portion 6, an operation portion side channel opening portion 9 functioning as one opening of a channel to be penetrated through the insertion portion 3, and a straight/side varying lever 10 at its proximal end portion.

The image catheter 2 is inserted to the channel in the insertion portion 3 from the operation portion side channel opening portion 9 of the operation portion 4, and the distal end surface 11 is arranged at the distal end portion 5. In addition, the distal end portion 5 is provided with an A/W (air/water) nozzle 5a for blowing a fluid or a fluid of a gaseous matter against the distal end surface 11 of the image catheter 2.

As shown in FIGS. 20 and 21, the distal end portion 5 is a toric member in which a groove portion 5a from the distal end surface towards the proximal end direction is formed such that a part of an outer circumferential part is notched substantially in the center. The groove portion 5a of the distal end portion 5 is provided with a raising table 20 substantially in a trapezoid shape in a cross section, which configures a varying mechanism.

The distal end portion 5 has a stopper 16 substantially in a columnar shape extended with the outer peripheral surface fixed in position to the proximal end of the groove portion 5a. In addition, the distal end portion 5 is provided with the endoscope channel 17 having the distal end surface fixed in position to the proximal end of the groove portion 5a. The endoscope channel 17 is composed of a flexible tube, and its proximal end portion is coupled to the operation portion side channel opening portion 9 of the operation portion 4. That is, the image catheter 2 introduced from the operation portion side channel opening portion 9 is penetrated through the endoscope channel 17.

Into the raising table 20 provided at the distal end portion 5, a pin 25 is penetrated in the lower end part here on the other side of a side straight varying surface 20a, that will be described later, in the up and down directions as seen towards the paper surface of FIG. 21. The raising table 20 is arranged so as to freely turn with respect to the distal end portion 5 about the axis of a pin 25. Also, the pin 25 is fixed to a turning support portion 5b provided to a wall surface of the groove portion 5a of the distal end portion 5 at both end portions.

The raising table 20 includes the side straight varying surface 20a that configure its one surface and a scope arrangement hole 21 with a hole diameter set as, for example, about 2 to 3 mm, penetrating substantially the center. The side straight varying surface 20a is provided with at its substantial center an opening portion 21a of the scope arrangement hole 21, and in the circumference of the opening portion 21a, four optical members 22 functioning as illumination windows according to the present embodiment. It should be noted that the distal end part of the image catheter 2 perpetrating through the endoscope channel 17 is penetrated through and arranged in the scope arrangement hole 21.

In addition, the raising table 20 is provided with the LEDs 23 for emitting illumination light inside of the vicinity of the four optical members 22 on the back side. The LEDs 23 are respectively electrically connected to cables 24a accommodated in an electric cable 24. The electric cable 24 is supplied with electricity via the insertion portion 3, the operation portion 4, and the universal cable 4a for causing the LED 23 to emit light from the control device. It should be noted that according to the present embodiment, the LED 23 for emitting illumination light is used, but an optical irradiation member such as a light guide may be used instead.

Furthermore, the raising table 20 has a long hole 26 on the upper side from the vicinity of the side straight varying surface 20a to the proximal end in the up and down directions as seen towards the paper surface of FIG. 21. Into the long hole 26, a slide pin 27 is penetrated so as to freely slide. One end portion of an operation wire 15 is coupled to the slide pin 27.

It should be noted that the other end portion of the operation wire 15 is coupled to a pulley not shown that is in motion in accordance with the turning operation on the straight/side varying lever 10 provided at the operation portion 4. As a result, the operation wire 15 is pulled or relaxed when the straight/side varying lever 10 is turned and operated in accordance with the turning of the pulley. In addition, the raising table 20 has a bent surface 28 formed with the proximal end upper portion of the scope arrangement hole 21 being bent at a predetermined curvature.

In the raising table 20 of the distal end portion 5 configured as in the above-mentioned manner, as shown in FIGS. 22 and 23, when the straight/side varying lever 10 of the operation portion 4 is turned and operated in a predetermined way, the operation wire 15 is pulled to be turned and operated about the axis of the pin 25.

To be more specific, in the raising table 20, the end part on the upper side as seen towards the paper surface of FIG. 21 is pulled in the proximal end direction by the pulled operation wire 15, thereby turning in the left direction about the axis of the pin 25. At this time, the slide pin 27 to which a distal end of the operation wire 15 is coupled is slid from one end to the other end of the long hole 26.

The turning of the raising table 20 is restricted as the surface coupled to the operation wire 15 abuts against the stopper 16. In other words, the facing direction of the side straight varying surface 20a of the raising table 20 is changed by about 90 degrees of the side surface direction of FIG. 22 from the distal end direction of the distal end portion 5 of FIG. 21 (to the upper side towards the paper surface of FIG. 22).

In this state, regarding the image catheter 2, while keeping the state in which the distal end part is arranged at the scope arrangement hole 21 of the raising table 20, the raising table 20 is turned by about 90 degrees, and therefore the direction in which the distal end surface 11 faces, which is the view direction, is changed by about 90 degrees.

That is, in the state shown in FIG. 21, the direction in which the distal end surface 11 of the image catheter 2 faces (the view direction) is in the same direction as the longitudinal direction of the insertion portion 3. On the other hand, in the state shown in FIG. 22, the turning operation is performed so that the side straight varying surface 20a of the raising table 20 is changed by about 90 degrees, the direction in which the distal end surface 11 of the image catheter 2 faces (the view direction) is substantially perpendicular to the axis of the longitudinal direction of the insertion portion 3.

As a result, the view direction of the image catheter 2 can be changed by a varying (switching) mechanism portion configured by the respective components such as the raising table 20 described above, to the straight vision state direction that is the same direction as the direction in which the distal end surface of the distal end portion 5 of the insertion portion 3 faces, and to the lateral vision state direction that is the direction substantially perpendicular to the direction in which the distal end surface of the distal end portion 5 of the insertion portion 3 faces.

Also, the irradiation direction of the LED 23 for irradiation, which is arranged at the raising table 20, is changed to the above-mentioned straight vision state direction and lateral vision state direction in accordance with the direction in which the side straight varying surface 20a faces.

The endoscope 1 configured as in the above-mentioned manner according to the present embodiment is inserted while keeping the straight visual state (V1 direction in the drawing) where the view direction that is the direction in which the distal end surface 11 of the image catheter 2 faces is the same direction as the proceeding direction of the insertion portion 3, when the insertion portion 3 is inserted to the duodenum 50 as shown in FIG. 24. In this state, the operator can visually check the insert direction of the insertion portion 3 through an image of the image catheter 2, and therefore in accordance with the bending body cavity, the insertion portion 3 of the endoscope 1 is subjected to a twist operation or the like, whereby the insert to the duodenum 50 can be facilitated.

Then, when the endoscope 1 reaches, as shown in FIG. 25, the vicinity of a duodenal papilla portion (hereinafter, simply referred to as papilla portion) 51 which is an entrance and an exit of the bile duct 52 and the pancreatic duct 53, as described above, the view direction that is the direction in which the distal end surface 11 of the image catheter 2 faces is changed by about 90 degrees from the straight vision state to the lateral vision state (V2 direction in the drawing). In this state, as the operator can visually check the body cavity wall of the duodenum 50 through the image of the image catheter 2, it is possible to easily discover the papilla portion 51 on the body cavity wall of the duodenum 50. In addition, the operator can easily identify the position and the size of an abnormal area of the body cavity wall by changing the view direction of the endoscope 1 to the lateral vision state.

As the result, the endoscope 1 according to the present embodiment has a configuration where the view direction can be easily changed to the straight vision state in which the insertion portion 3 is inserted into the body cavity up to a certain target object on the body cavity wall (the papilla portion 51 in the above-mentioned description) and to the lateral vision state in which the target area is discovered, and the position and the size are identified.

It should be noted that the configuration of the distal end portion 5 having the varying (switching) mechanism for changing the view direction of the image catheter 2 to the straight vision state or the lateral vision state may be a configuration described hereinafter.

As shown in FIGS. 26 and 27, the distal end portion 5 of the endoscope 1 according to a modification example is provided with an penetration hole 30 through which the scope insertion portion 12 of the image catheter 2 or the like extended from the endoscope channel 17 is penetrated.

The penetration hole 30 is branched off on the distal end side of the distal end portion 5 and one side surface side. One side of the penetration hole 30 opened on the distal end surface of the branched distal end portion 5 serves as a straight vision hole 31 and the other side thereof opened on one side surface of the distal end portion 5 serves as a lateral vision hole 32.

The distal end portion 5 is provided with the plural optical members 22 in the vicinity of the opening portions of the respective holes 31 and 32 on the distal end surface and on side surface, and irradiating LEDs 23a and 23b respectively on the back sides of the optical members 22.

Also, the distal end portion 5 has a concave portion 34 for accommodating a gate 35 that configures the substantially plate shaped varying mechanism on an inner surface of the penetration hole 30 on the opposite side of a part of the lateral vision hole 32 branching and extending. In the gate 35, one end portion on the proximal end side is turned and held by a pin 35a, and the other end portion on the distal end side is coupled to the distal end of the operation wire 15. The operation wire 15 is penetrated through the penetration hole 33 formed in the distal end portion 5, and as described above, is pulled and relaxed through the turning operation on the straight/side varying lever 10 of the operation portion 4.

The penetration hole 33 has a shape in which the distal end part is bent towards the penetration hole 30 side, and is opened at a part where the straight vision hole 31 and the lateral vision hole 32 are branched. In addition, the bent portion of the penetration hole 33 is provided with a bar member 33a that suppresses the friction on the contact surface due to pulling and relaxing of the operation wire 15.

Then, the distal end part of the gate 35 is pulled by the operation wire 15 pulled through the turning operation on the straight/side varying lever 10 of the operation portion 4, and is turned about the pin 35a in a raising direction.

As shown in FIG. 26, in the state where the gate 35 is accommodated in the concave portion 34, at the distal end portion 5, the scope insertion portion 12 of the image catheter 2 is inserted in the straight vision hole 31 while being in a substantially straight form. At this time, the image catheter 2 is arranged in the straight vision hole 31 such that the direction in which the distal end surface faces is the same direction as the direction in which the distal end surface of the distal end portion 5 faces. Therefore, the view direction of the image catheter 2 is in the straight vision state of the longitudinal axis direction of the insertion portion 3 of the endoscope 1.

On the other hand, as shown in FIG. 27, with the turning operation on the straight/side varying lever 10 of the operation portion 4, in the state in which the gate 35 is raised, in the distal end portion 5, the distal end part of the scope insertion portion 12 of the image catheter 2 is inserted to the lateral vision hole 32 while being bent along the gate 35. At this time, the image catheter 2 is arranged at the lateral vision hole 32 such that the direction in which the distal end surface thereof faces is the same as the direction in which one side surface of the distal end portion 5 faces, or the direction substantially perpendicular to the longitudinal axis. Therefore, the view direction of the image catheter 2 is in the lateral view state with the direction substantially perpendicular to the longitudinal axis direction of the insertion portion 3 of the endoscope 1.

It should be noted that in the case of the straight vision state, the electric power is supplied to the LEDs 23a corresponding to the respective optical members 22 arranged at the distal end surface of the distal end portion 5. In addition, in the case of the lateral vision state, the electric power is supplied to the LEDs 23b corresponding to the respective optical members 22 arranged at one side surface of the distal end portion 5. The electric power supply to the respective LEDs 23a and 23b is automatically switched by an external control device to be operated simultaneously in accordance with the turning operation on the straight/side varying lever 10 of the operation portion 4.

Furthermore, when the view direction of the image catheter 2 is desired to be changed to the straight vision state or the lateral vision state, the operator carries out the predetermined turning operation on the straight/side varying lever 10 of the operation portion 4 by pulling the image catheter 2 by a certain length to its hand side. With the operation on the straight/side varying lever 10, the gate 35 is raised or accommodated in the concave portion 34.

Then, after the operation on the straight/side varying lever 10, the operator can insert the distal end part of the scope insertion portion 12 of the image catheter 2 to the straight vision hole 31 when the gate 35 is accommodated in the concave portion 34 and to the lateral vision hole 32 when the gate 35 is raised. As a result, the operator can change the view direction of the image catheter 2 to the straight vision state or the lateral vision state.

In addition, in the distal end portion 5, the configuration of the distal end portion 5 having the varying mechanism for changing the view direction of the image catheter 2 to the straight vision state or the lateral vision state may be a protrusion portion 36 shown in FIGS. 28 and 29 instead of the gate 35.

To be more specific, as shown in FIGS. 28 and 29, the distal end portion 5 according to the present modification example includes a concave portion 38 for accommodating the protrusion portion 36 configuring the varying mechanism on the inner surface of the penetration hole 30 on the opposite side to a part of the lateral vision hole 32 branching and extending. The protrusion portion 36 is formed with an inclined surface and substantially has a triangular shape in cross section, and a spring 36a is fixed to the lower surface on the opposite direction side of the lateral vision hole 32. That is, the spring 36a biases the protrusion portion 36 towards the lateral vision hole 32 side.

In addition, the distal end portion 5 includes an penetration hole 39 through which the operation wire 15 is penetrated on the side on the concave portion 38 side and has a concave portion 39a with its side opened in the penetration hole 39. The concave portion 39a is formed on the inner side of the penetration hole 30 on the proximal end side of the concave portion 38.

The distal end part of the operation wire 15 is coupled to a slide board 37. The slide board 37 is a board member substantially having an L-shape in cross section, and a spring 37a is fixed to a rear surface serving as the distal end side of the distal end portion 5 at the bent part. The bent part of the slide board 37 is accommodated in the concave portion 39a together with the spring 37a. In addition, the spring 37a biases the slide board 37 forward that is the distal end side of the distal end portion 5.

Then, as shown in FIG. 28, with the bias force of the spring 37a, the slide board 37 blocks the opening portion of the concave portion 38 with its board surface in the state in which the operation wire 15 is not pulled. At this time, as the protruding end on the lateral vision hole 32 side abuts the slide board 37, the protrusion portion 36 is accommodated in the concave portion 38.

In this state, in the distal end portion 5, the scope insertion portion 12 of the image catheter 2 is inserted to the straight vision hole 31 as being substantially in a straight shape. At this time, the image catheter 2 is arranged at the straight vision hole 31 such that the direction in which the distal end surface faces is the same as the direction in which the distal end surface of the distal end portion 5 faces. Therefore, the view direction of the image catheter 2 is in the straight vision state in the longitudinal axis direction of the insertion portion 3 of the endoscope 1.

On the other hand, as shown in FIG. 29, the operation wire 15 is pulled through the turning operation on the straight/side varying lever 10 of the operation portion 4, the slide board 37 is pulled to the rear side, and the concave portion 38 is opened. At this time, the protrusion portion 36 protrudes, while receiving the bias force of the spring 36a, from the concave portion 38 into the penetration hole 30 on the lateral vision hole 32 side.

In this state, in the distal end portion 5, the distal end part of the scope insertion portion 12 of the image catheter 2 is inserted to the lateral vision hole 32 in the state of being bent along the inclined surface of the protrusion portion 36. At this time, the image catheter 2 is arranged at the lateral vision hole 32 such that the direction in which the distal end surface faces is the same as the direction in which one side surface of the distal end portion 5 faces, or the direction substantially perpendicular to the longitudinal axis. Therefore, the view direction of the image catheter 2 is in the lateral vision state in the direction substantially perpendicular to the longitudinal axis direction of the insertion portion 3 of the endoscope 1.

In addition, when the view direction of the image catheter 2 is desired to be changed to the straight vision state or the lateral vision state, the operator carries out the predetermined turning operation on the straight/side varying lever 10 of the operation portion 4 by pulling the image catheter 2 by a certain length to its hand side. It should be noted that when the slide board 37 moves to the front side, the protrusion portion 36 protruding into the penetration hole 30 is pushed into the concave portion 38 as the distal end part of the slide board 37 abuts the inclined surface of the protrusion portion 36. After that, the slide board 37 closes the opening portion of the concave portion 38, whereby the protrusion portion 36 is accommodated in the concave portion 38.

Therefore, after the operation on the straight/side varying lever 10, the operator can insert the distal end part of the scope insertion portion 12 of the image catheter 2 to the straight vision hole 31 when the protrusion portion 36 is accommodated in the concave portion 38, and to the lateral vision hole 32 when the protrusion portion 36 protrudes towards the penetration hole 30. As a result, the operator can change the view direction of the image catheter 2 to the straight vision state or the lateral vision state.

It should be noted that the protrusion of the protrusion portion 36 and the accommodation into the concave portion 38 may be performed, for example, with use of an electromagnetic solenoid.

With the configurations of the distal end portion 5 according to the above-mentioned respective modification examples, the same effect of the present embodiment can be achieved.

Furthermore, the endoscope 1 provided with the configuration having the distal end portion 5 with the varying mechanism for changing the above-mentioned view direction to the straight vision state or the lateral vision state, as shown in FIG. 30, can also be used by inserting the guide catheter 40 through which the image catheter 2 is penetrated according to the first embodiment into the endoscope channel 17 for a surgery based on the above-mentioned endoscopic retrograde pancreatico-cholangiography.

### (Third Embodiment)

Next, a third embodiment useful for understanding but not part of the invention will be described. According to the present embodiment, as one configuration of the endoscopic system 18, the guide catheter 40 used in combination with the endoscope 1 according to the first embodiment, to which a characteristic configuration is added, will be hereinafter described.

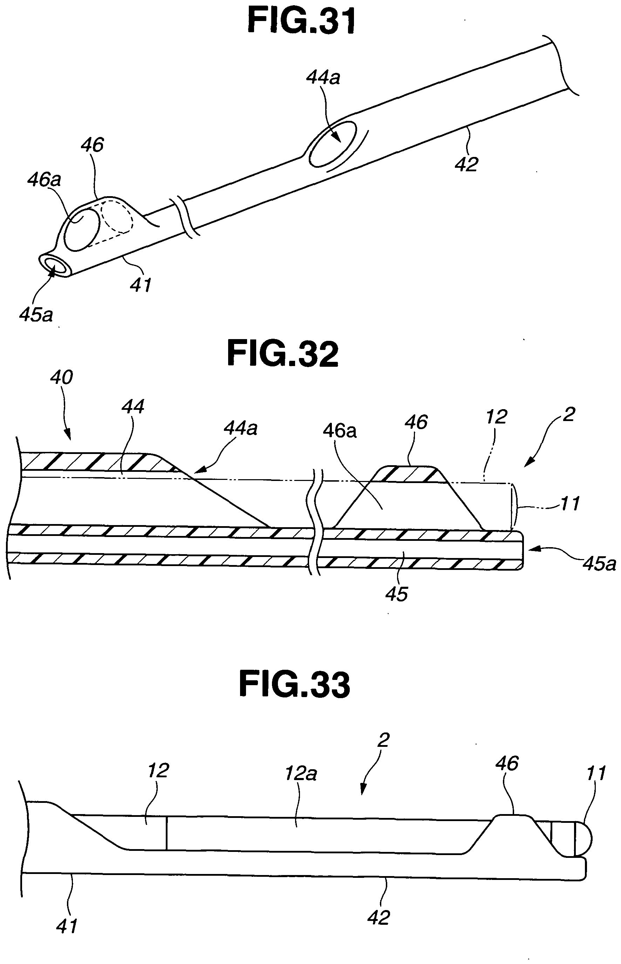

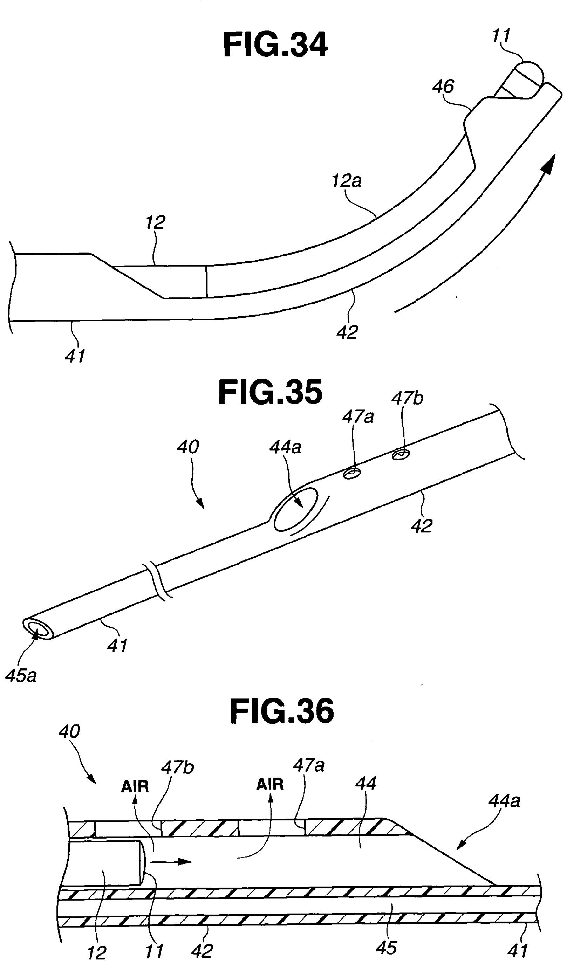

FIGS. 31 to 34 represent the third embodiment of the present invention: FIG. 31 shows a configuration of a distal end part of the guide catheter 40, FIG. 32 is a cross sectional view of the distal end part of the guide catheter 40, FIG. 33 is a side view of the distal end part of the guide catheter 40 in a state in which the image catheter provided with a bending portion where the distal end part is held by a holding portion is penetrated, and FIG. 34 is a side view of the distal end part of the guide catheter in a state in which the image catheter is penetrated, representing a state in which the image catheter is bent from the state in FIG. 33. It should be noted that in the description on the present embodiment, the same reference numerals are used for the configurations described in the above-mentioned embodiments and the detail description will be omitted.

As shown in FIGS. 31 and 32, the guide catheter 40 according to the present embodiment includes a holding portion 46 capable of elastically deforming with a monorail configuration at the distal end part on the extending side of the guide portion 41. The holding portion 46 has a hole portion 46a to which the distal end part of the image catheter 2 or various treatment instruments can be inserted and detached.

The holding portion 46 is arranged in a direction protruding from the side surface portion of the guide portion 41 so as to be on the hole axis side of the scope/treatment instrument channel 44 provided within the guide catheter insertion portion 42. In addition, the hole portion 46a of the holding portion 46 has a hole axis substantially parallel to the hole axis of the scope/treatment instrument channel 44 when the guide portion 41 is substantially in the straight state.

In this way, by providing the guide portion 41 with the holding portion 46, the guide catheter 40 can be held, as shown in FIG. 32, herein, as the scope insertion portion 12 of the image catheter 2 is inserted to the hole portion 46a of the holding portion 46.

Also, when the image catheter 2 or various treatment instruments is pulled to the proximal end side which is the operator's hand side, the scope insertion portion 12 or a sheath can be easily removed from the hole portion 46a of the holding portion 46. As a result, the operator can perform insertion and removal of the image catheter 2 or various treatment instruments with respect to the guide catheter 40, and can also cause various treatment instruments to function.

As described above, the guide catheter 40 according to the present embodiment can keep the state in which the distal end part of the image catheter 2 or various treatment instruments inserted to the scope/treatment instrument channel 44 is held at the holding portion 46 with reliability. For that reason, the operator can easily insert the guide catheter 40 as well as the image catheter 2 or various treatment instruments from the operation portion side channel opening portion 9 of the endoscope 1 to the endoscope channel 17 without shifting at the same time. In addition, the image catheter 2 or various treatment instruments being held at the holding portion of the guide portion 41, the operator can also easily insert the image catheter 2 or various treatment instruments from the papilla portion 51 to the bile duct 52 or the pancreatic duct 53.

It should be noted that in a case where the image catheter 2 is provided with a function of actively bending at the distal end part, as shown in FIG. 33, if such a state is effected that the distal end part of the image catheter 2 is penetrated and held by the holding portion 46 of the guide portion 41, the guide catheter 40 according to the present embodiment has such a configuration, as shown in FIG. 34, that the guide portion 41 is also bent while following the bending movement by a bending portion 12a of the image catheter 2. As a result, the operator can freely change the guide catheter 40 in a desired introducing direction by bending the bending portion 12a of the image catheter 2 as well as the guide portion 41 of the guide catheter 40.

### (Fourth Embodiment)

Next, a fourth embodiment useful for understanding but not part of the invention will be described. According to the present embodiment as well, the guide catheter 40 used in combination with the endoscope 1 as one configuration of the endoscopic system 18 according to the first embodiment, to which a characteristic configuration is added, will be described hereinafter.

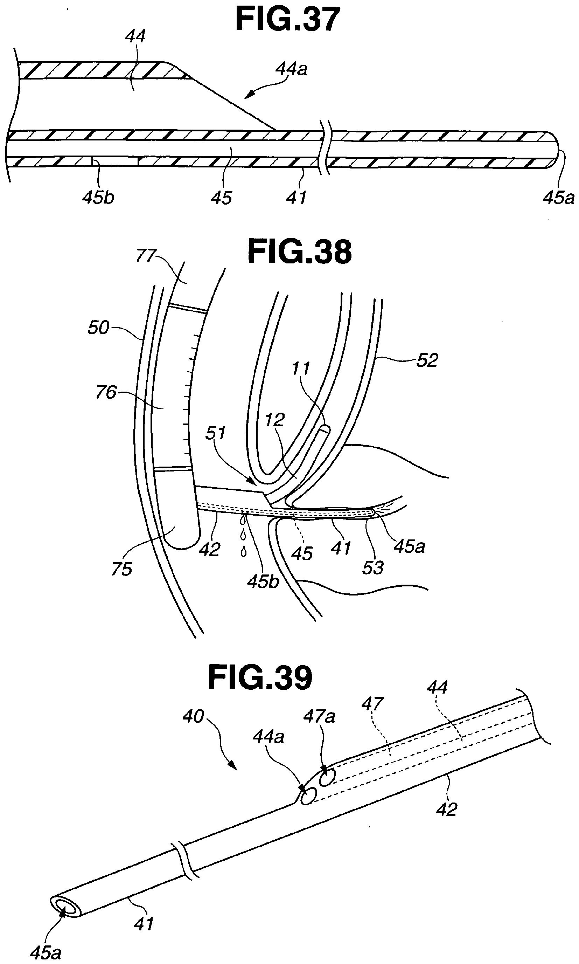

FIGS. 35 to 38 represent the fourth embodiment: FIG. 35 shows a configuration of a distal end part of the guide catheter 40, FIG. 36 is a cross sectional view of the distal end part of the guide catheter 40, FIG. 37 is a cross sectional view of the distal end part of the guide catheter 40 which is an modification example, and FIG. 38 is an explanatory diagram for an action of the guide catheter 40 of FIG. 37. It should be noted that in the description on the present embodiment, the same reference numerals are used for the configurations described in the above-mentioned embodiments and the detail description will be omitted.

As shown in FIGS. 35 and 36, the guide catheter 40 according to the present embodiment has plural, herein, two hole portions the hole portions 47a and 47b (configuring a first hole portion) bored and formed in the circumferential surface portion of the distal end part of the guide catheter insertion portion 42. The hole portions 47a and 47b allow the scope/treatment instrument channel 44 arranged at the guide catheter insertion portion 42 to communicate with the outside of the guide catheter 40.

It should be noted that the number of the hole portions 47a and 47b is not limited to two, and may be one or two or larger, and in the bile duct 52, the holes are preferably arranged at the distal end part of the guide catheter insertion portion 42 where no insertion is caused normally.

Incidentally, for example, in the state in which the opening portion 44a of the scope/treatment instrument channel 44 is inserted into the bile duct 52 or the pancreatic duct 53, when the image catheter 2 is inserted to the guide catheter 40, the air inside the scope/treatment instrument channel 44 is swept by the image catheter 2, whereby the air is discharged from the opening portion 44a into the bile duct 52 or the pancreatic duct 53. The air inside the bile duct 52 or the pancreatic duct 53 becomes a black shadow during the X-ray photography, so it becomes difficult for the operator to perform a judgment as to whether the black shadow is the gallstone 54 or the abnormal area.

The guide catheter 40 configured as in the above-mentioned manner according to the present embodiment is inserted and removed, as shown in FIG. 35, with respect to the scope/treatment instrument channel 44. Herein, the air swept by the scope/treatment instrument channel 44 from the proximal end side with the scope insertion portion 12 of the image catheter 2 can be discharged from the hole portions 47a and 47b. It is needless to mention that in various treatment instruments as well, in the guide catheter 40, the air swept from the proximal end side to the scope/treatment instrument channel 44 can be discharged from the hole portions 47a and 47b.

For that reason, the guide catheter 40 can prevent the air swept from the proximal end side into the scope/treatment instrument channel 44 from flowing into the bile duct 52 or the pancreatic duct 53 in the state where the opening portion 44a of the scope/treatment instrument channel 44 is inserted into the bile duct 52 or the pancreatic duct 53, even when the image catheter 2 or various treatment instruments are inserted to the scope/treatment instrument channel 44.

As a result, it is easier for the operator to perform the judgment on the gallstone 54 or the abnormal area during the X-ray photography as the black shadow caused by the air is not displayed.

Furthermore, with the above-mentioned configuration, the guide catheter 40 can also prevent sucking the contrast agent injected into the bile duct 52 or the pancreatic duct 53 in the state in which the opening portion 44a of the scope/treatment instrument channel 44 is inserted into the bile duct 52 or the pancreatic duct 53 when the image catheter 2 or various treatment instruments are removed from the scope/treatment instrument channel 44.

It should be noted that as shown in FIG. 37, a hole portion 45b (configuring a second hole portion) in communication with the fluid supply channel 45 opened in the guide portion 41 of the guide catheter 40 may be provided on the guide catheter insertion portion 42. It is desirable that the hole portion 45b is arranged on the guide catheter insertion portion 42 in the vicinity of the guide portion 41. In addition, the number of the hole portion 45 is not limited to one but may be plural.

In the guide catheter 40 configured in this manner, as shown in FIG. 38, for example, the guide portion 41 is inserted into the pancreatic duct 53, and the pancreatic fluid within the pancreatic duct 53 can be discharged from the hole portion 45b formed on the guide catheter insertion portion 42 to the duodenum 50.

### (Fifth Embodiment)

Next, a fifth embodiment of the present invention will be described. According to the present embodiment as well, as one configuration of the endoscopic system 18, the guide catheter 40 according to the first embodiment used in combination with the endoscope 1, to which a characteristic configuration is added, will be described hereinafter. It should be noted that in the description on the present embodiment, the same reference numerals are used for the configurations described in the above-mentioned embodiments and the detail description will be omitted.

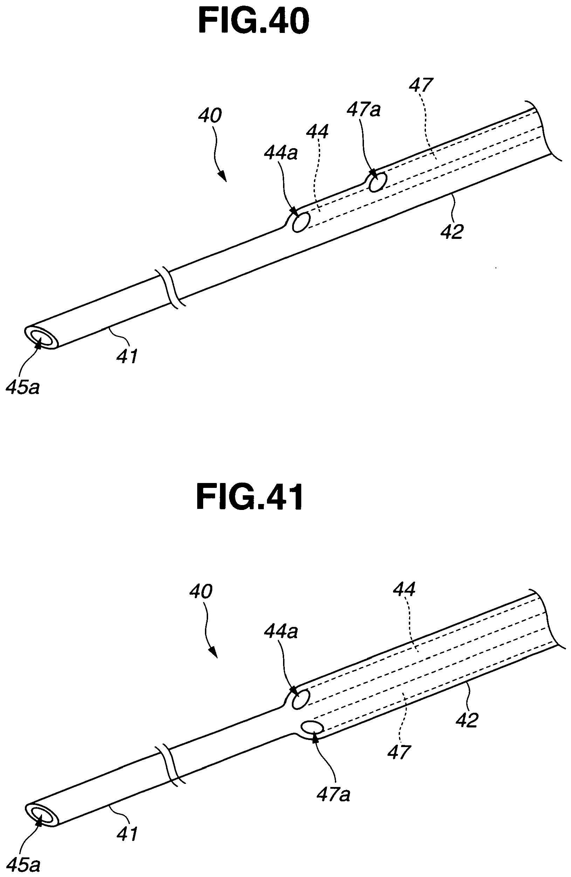

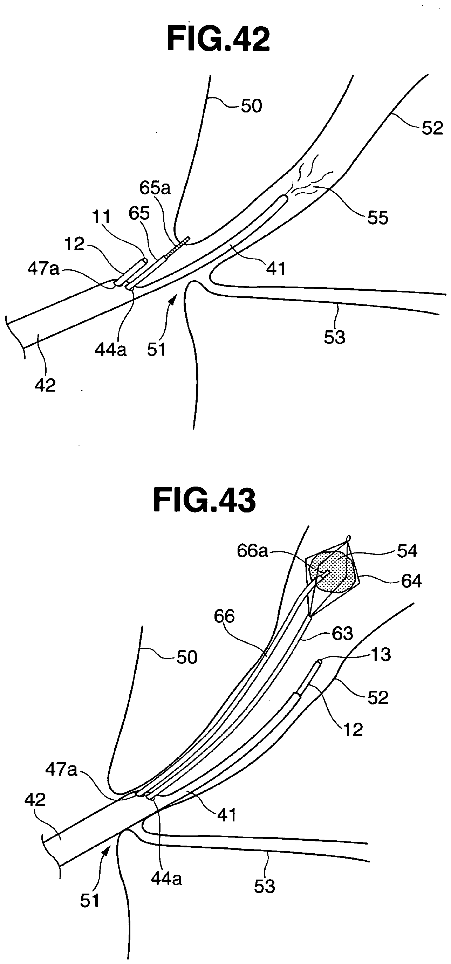

FIGS. 39 to 43 represent the fifth embodiment of the present invention: FIG. 39 is a perspective view of the distal end part of the guide catheter, FIG. 40 is a perspective view of the distal end part of the guide catheter, representing a first modification example, FIG. 41 is a perspective view of the distal end part of the guide catheter, representing a second modification example, FIG. 42 illustrates a use example of the guide catheter of FIG. 39, and FIG. 43 illustrates a use example of the guide catheter of FIG. 39.

As shown in FIG. 39, the guide catheter 40 according to the present embodiment includes, in addition to the opening portion (hereinafter, referred to as first opening portion) 44a of the treatment instrument channel (hereinafter, referred to as the first treatment instrument channel) 44, an opening portion (hereinafter, referred to as second opening portion) 47a at the distal end of the guide catheter insertion portion 42 which is a root portion of the guide portion 41, and a treatment instrument channel (hereinafter, referred to as second treatment instrument channel) 47 at the guide catheter insertion portion 42 from the opening portion 47a which is separately arranged from the treatment instrument channel 44.

It should be noted that regarding the guide catheter 40, with respect to the position of the first opening portion 44a, the second opening portion 47b may be at a position, as shown in FIG. 40, which is shifted in the long axis direction of the guide catheter insertion portion 42 or at a position, as shown in FIG. 41, on the opposite side across the root portion of the guide portion 41.

Regarding the guide catheter 40 configured in this manner according to the present embodiment, as shown in FIG. 42, when the papilla portion of the duodenum 50 is cut with the needle-shaped knife 65 in a state in which only the guide portion 41 is inserted into the bile duct 52, for example, the operator inserts the needle-shaped knife 65 which is the treatment instrument to the first treatment instrument channel 44 to allow the knife portion 65a to extend from the first opening portion 44a and also the scope insertion portion 12 of the image catheter 2 is inserted to the second treatment instrument channel 47 to allow the distal end surface 11 to extend from the second opening portion 47b.

With use of the guide catheter 40 described above, on the basis of the video from the image catheter 2, the operator can carry out a procedure while visually checking an image close to the papilla portion 51 to be cut. Furthermore, the guide catheter 40 can obtain the close-up video based on the image catheter 2, which is useful for finding a lesion location in an early stage, and the treatment can be conducted by using other treatment instrument without changing the state.

Also, the operator can inject the contrast agent 55 from the opening portion 45a at the distal end of the guide portion 41 into the bile duct 52. It should be noted that the guide portion 41 is not limited to the insertion into the bile duct 52 but of course may be inserted into the pancreatic duct 53.

In addition, in a state in which the distal end part of the guide catheter insertion portion 42 of the guide catheter 40 according to the present embodiment is inserted into the bile duct 52, as the distal end surface 11 of the image catheter 2 is extended from 45a the opening portion 45a at the distal end of the guide portion 41, the operator distinguishes a color of the gallstone 54, so that types of the gallstone 54 can be determined, for example, as a relatively rigid stone of cholesterol, calcium, or the like in the case of a white color or a relatively flexible stone of bilirubin or the like in the case of a brown color.

For that reason, not only the operator can select the treatment instrument to be used depending on the type of the gallstone 54, but also as shown in FIG. 43, for example, causes the basket grasping forceps 63 to be extended from the first opening portion 44a via the first treatment instrument channel 44, to capture and grasp the gallstone 54 within the basket wire 64, and also causes an electrohydraulic shock wave lithotripsy apparatus 66 which is a treatment instrument to be extended from the opening portion 47a via the second treatment instrument channel 47, to place a shock wave generation portion 66a on the gallstone 54 grasped by the basket wire 64. In this way, the operator can also conduct the treatment for pulverizing the gallstone 54. It should be noted that the electrohydraulic shock wave lithotripsy apparatus 66 is a treatment instrument for pulverizing the stone by way of a compression wave (shock wave).

### (Sixth Embodiment)

Next, a sixth embodiment useful for understanding but not part of the invention will be described. According to the present embodiment as well, as one configuration of the endoscopic system 18, the guide catheter 40 according to the first embodiment used in combination with the endoscope 1, to which a characteristic configuration is added, will be described hereinafter.

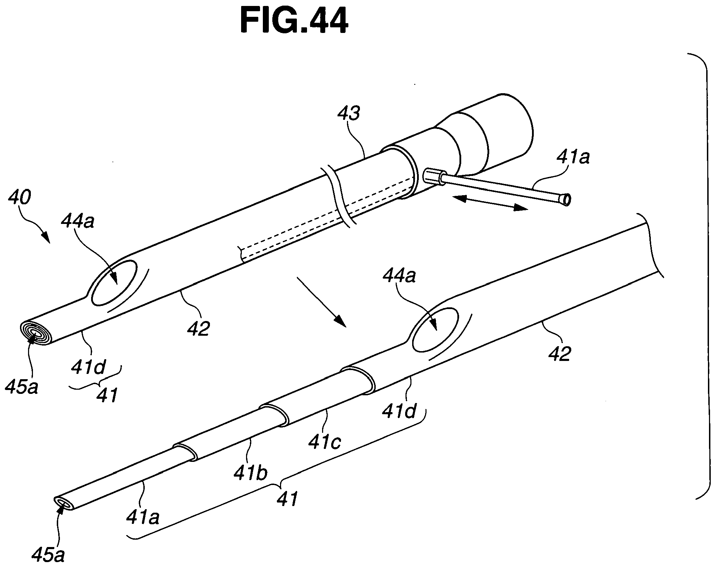

FIG. 44 represents the sixth embodiment useful for understanding but not part of the invention, showing a configuration of the distal end part of the guide catheter 40. It should be noted that in the description on the present embodiment as well, the same reference numerals are used for the configurations described in the above-mentioned embodiments and the detail description will be omitted.

As shown in FIG. 44, the guide catheter 40 according to the present embodiment has the configuration for expanding and contracting to vary the length of the guide portion 41. To be more specific, the guide portion 41 has plural, herein, three tube bodies 41a to 41c and a substantially cylindrical extension portion 41d extending from the peripheral portion of the distal end surface of the guide catheter insertion portion 42.

The guide portion 41 is located at the innermost portion of the extension portion 41d when being in the contracted state. The outer diameter of the tube body 41a located at the most distal end when being in the state where the guide portion 41 is extended the tube body 41a is set to the smallest diameter, and gradually, the respective outer diameter of the tube body 41a, the tube body 41b, and the tube body 41c are set to have larger diameters in this order.

The tube bodies 41a to 41c are accommodated in the extension portion 41d from the innermost portion in the order of the tube body 41a, the tube body 41b, and the tube body 41c when the guide portion 41 is in the contracted state.

In other words, in the guide portion 41, the outer diameter of the tube body 41a is substantially the same as the hole diameter of the tube body 41b, the outer diameter of the tube body 41b is substantially the same as the hole diameter of the tube body 41c, and the outer diameter of the tube body 41c is substantially the same as the hole diameter of the extension portion 41d. Therefore, the guide portion 41 can extend and contract in the predetermined length while the respective tube bodies 41a to 41c are slid back and forward.

Also, the respective tube bodies 41a to 41c and the extension portion 41d are provided with a stopper not shown in the drawing, for limiting the sliding range and allowing the guide portion 41 to engage the respective parts in the maximum length or the minimum length.

It should be noted that the tube body 41a includes the fluid supply channel 45 for injecting the contrast agent or the like, arranged so as to freely slide from the extension portion 41d within the guide catheter insertion portion 42 with the proximal end portion extending from the side peripheral portion of the treatment instrument penetration portion 43 located at the proximal end of the guide catheter 40.

The extension and contraction operation on the guide portion 41 is performed by pulling and relaxing the proximal end part of the tube body 41a. In addition, the operator can inject a fluid like a contrast agent or the like from the opening portion 45a via the fluid supply channel 45 to the bile duct 52 or the pancreatic duct 53 by connecting the syringe or the like to the proximal end part of the tube body 41a.

As the guide catheter 40 configured as in the above can vary the length of the guide portion 41 in accordance with the duct diameter, length, and shape of the bile duct 52 or the pancreatic duct 53 which vary depending on a subject, it is possible to enhance the insertability of the image catheter 2 and various treatment instruments from the papilla portion 51 to the bile duct 52 or the pancreatic duct 53.

### (Seventh Embodiment)

Next, a seventh embodiment of the present invention will be described. According to the present embodiment as well, as one configuration of the endoscopic system 18, the guide catheter 40 used in combination with the endoscope according to the first embodiment, to which a characteristic configuration is added, will be described hereinafter.

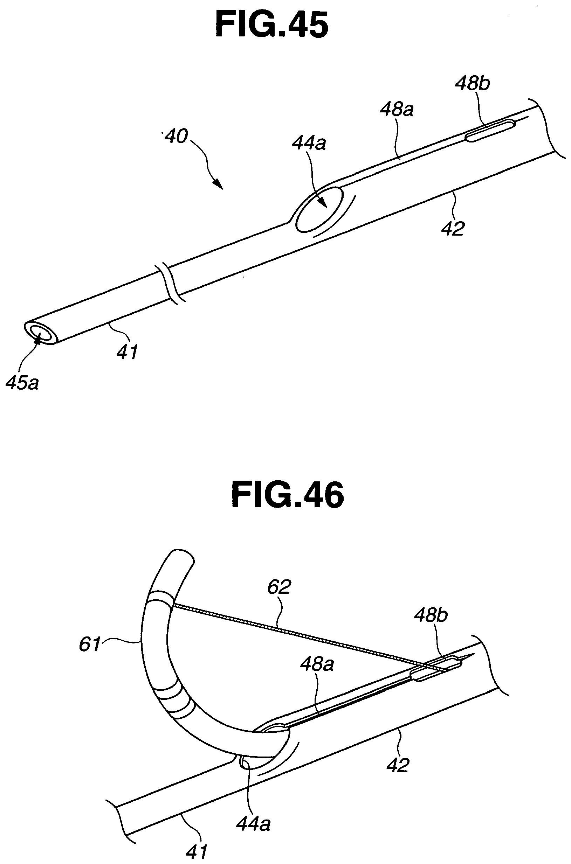

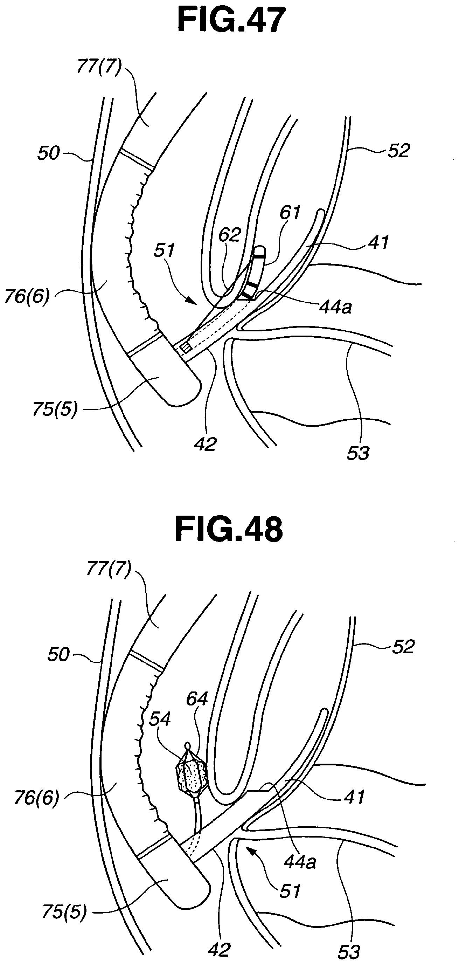

FIGS. 45 to 48 represent the seventh embodiment of the present invention: FIG. 45 shows a configuration of a distal end part of the guide catheter, FIG. 46 shows the distal end part of the guide catheter in a state in which a distal end part of a papillotomy knife inserted to this guide catheter is shaped arcuate, FIG. 47 is an explanatory diagram for an operation of cutting the duodenal papilla with the papillotomy knife, and FIG. 48 shows a state in which basket grasping forceps grasping a gallstone are pulled to the duodenum. It should be noted that in the description on the present embodiment as well, the same reference numerals are used for the configurations described in the above-mentioned embodiments and the detail description will be omitted.

As shown in FIG. 45, the guide catheter 40 according to the present embodiment has a slit 48a and a long hole 48b at the distal end part of the guide catheter insertion portion 42.

The slit 48a is formed with a predetermined length from the distal end of the peripheral portion of the guide catheter insertion portion 42 on the opposite side where the guide portion 41 extends, along the axis of the guide catheter insertion portion 42. Then, the long hole 48b is formed at the distal end part of the slit 48a.

As described in the first embodiment, the papillotomy knife 61 inserted to the guide catheter 40 is formed into an arcuate shape for cutting the papilla portion 51 as the wire cutter 62 of the distal end part is pulled.

In the guide catheter 40 according to the present embodiment, as shown in FIG. 46, when the papillotomy knife 61 is formed into an arcuate shape, even in a case where the entire wire cutter 62 does not extend from the distal end of the guide catheter insertion portion 42, the wire cutter 62 is passed through the guide catheter insertion portion 42 with use of the slit 48a. For that reason, the wire cutter 62 of the papillotomy knife 61 can be easily and reliably in tension without the disturbance of the guide catheter insertion portion 42.

To be still more specific, as shown in FIG. 47, even in a state in which the guide portion 41 of the guide catheter 40 is inserted from the papilla portion 51 into the bile duct 52 and also the distal end of the guide catheter insertion portion 42 is inserted to the papilla portion 51, when the operator pulls the wire cutter 62, the papillotomy knife 61 can be easily shaped arcuate.

At this time, such a state is obtained that a part of the wire cutter 62 protrudes from the distal end part of the guide catheter insertion portion 42 via the slit 48a. Then, the operator performs cutting of the papilla portion 51 by applying the high frequency to the wire cutter 62.

Furthermore, as one end part of the wire cutter 62 supported by the papillotomy knife 61 at the proximal end side is located at the long hole 48b, the operator can perform the turning operation on the papillotomy knife 61 in the back and forward directions and about the axis by the shape of the long hole 48b.

In consequence, the operator can perform the operation at a suitable position where the papillotomy knife 61 cuts the papilla portion 51, while the distal end of the guide catheter insertion portion 42 abuts the papilla portion 51, without moving the guide catheter 40.

It should be noted that with the guide catheter 40 having the slit 48a and the long hole 48b, not only the operability enhancement of the papillotomy knife 61 can be achieved, but also, for example, as shown in FIG. 48, the basket wire 64 of the basket grasping forceps 63 can protrude from the distal end part of the guide catheter insertion portion 42. With this configuration, it is easier for the operator to expose the basket wire 64 in the duodenum 50 even when the distal end part of the guide catheter insertion portion 42 is in the vicinity of the papilla portion 51.

In other words, when the gallstone or the pancreatic stone is picked up, even in the case where the distal end of the guide catheter insertion portion 42 of the guide catheter 40 is inserted to the papilla portion 51, the operator can expose the basket wire 64 of the basket grasping forceps 63 from the slit 48a and the long hole 48b of the guide catheter 40.

### (Eighth Embodiment)

Next, an eighth embodiment useful for understanding but not part of the invention will be described. According to the present embodiment, cross sectional shapes of the endoscope channel 17, the guide catheter 40, the image catheter 2 and various treatment instruments of the endoscope 1 to which a characteristic configuration is added will be described hereinafter.

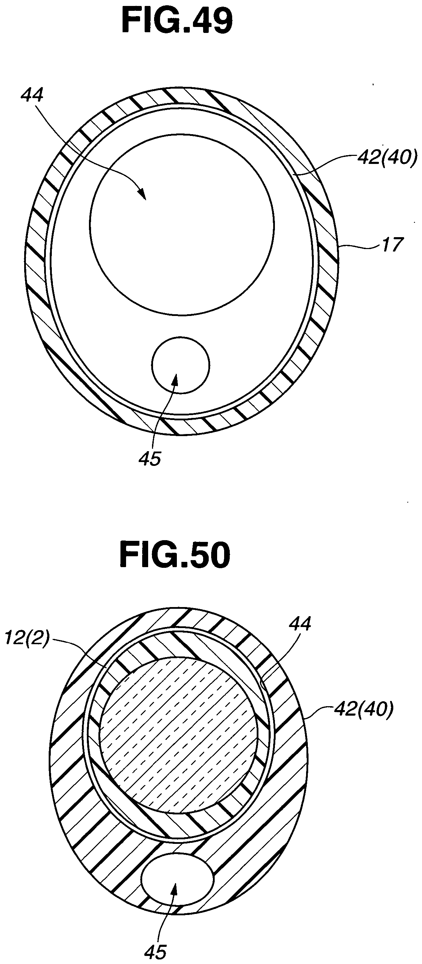

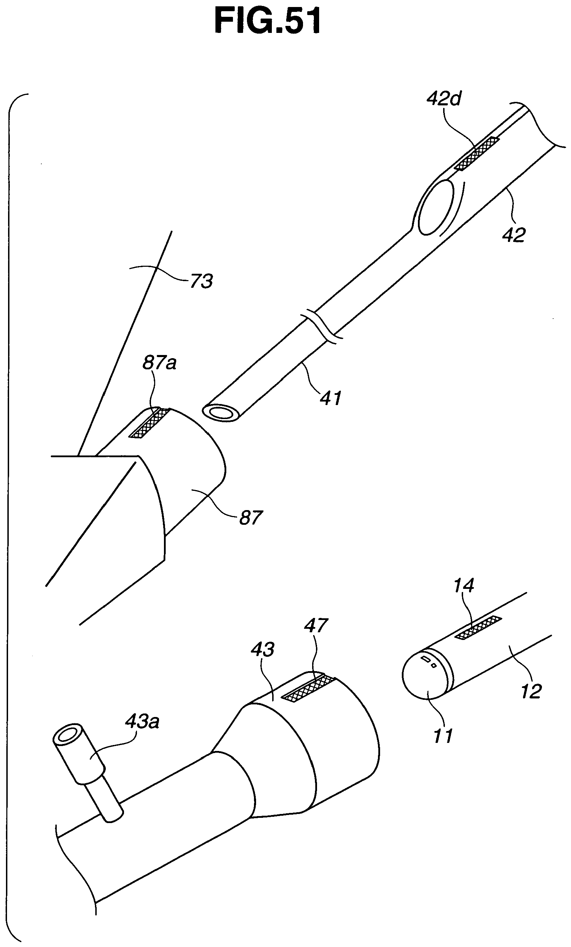

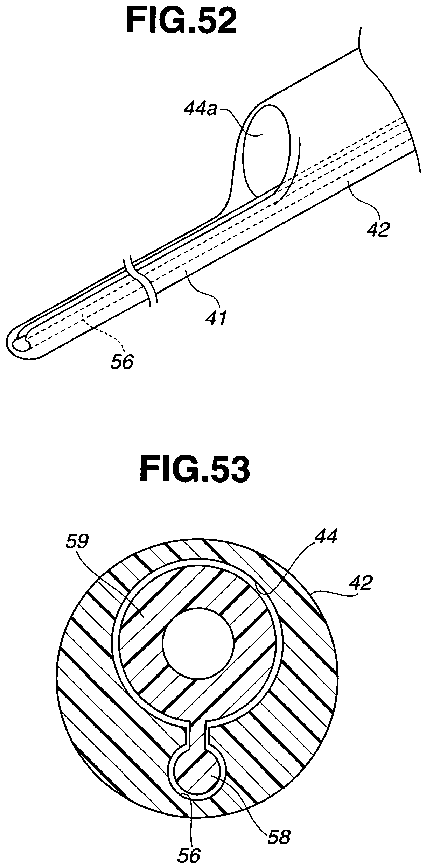

FIGS. 49 to 53 represent the eighth embodiment: FIG. 49 is a cross sectional view of an endoscope channel and a guide catheter penetrated through this endoscope channel, FIG. 50 is a cross sectional view of the guide catheter and an image catheter penetrated through this guide catheter, FIG. 51 illustrates indices for respectively regulating an inserting direction about a long axis on a channel opening portion on an operation portion side of the lateral vision endoscope, the distal part of the guide catheter, a treatment instrument penetration portion of the guide catheter, and the distal part of the image catheter which is a treatment instrument, FIG. 52 illustrates a rail-shaped groove portion formed from the guide portion of the guide catheter to the proximal end of the guide catheter, and FIG. 53 is a cross sectional view of the guide catheter of FIG. 52 in a state in which a treatment instrument is penetrated. It should be noted that in the description on the present embodiment as well, the same reference numerals are used for the configurations described in the above-mentioned embodiments and the detail description will be omitted.

As shown in FIG. 49, the endoscope channel 17 according to the present embodiment provided to the endoscope 1 has an eclipse shape in the transverse cross section. The guide catheter 40 substantially has the same transverse cross sectional shape as the penetration path in the eclipse shape of the endoscope channel 17 through which the guide catheter insertion portion 42 is penetrated.

With the above-mentioned configuration, the turning of the guide catheter 40 is prohibited about the longitudinal axis in the endoscope channel 17, and the guide catheter 40 is stable in the endoscope channel 17. In addition, the guide catheter 40 has the improved operability as the shaking is prevented by the endoscope channel 17.

Furthermore, as shown in FIG. 50, in the guide catheter 40, the transverse cross sectional shape of the scope/treatment instrument channel 44 may be formed in eclipse. In addition, the scope insertion portion 12 of the image catheter 2 to be penetrated through this scope/treatment instrument channel has substantially the same transverse cross sectional shape as the penetration path in the eclipse shape of the scope/treatment instrument channel 44. It should be noted that while FIG. 50 shows the image catheter 2, a sheath of various treatment instruments has also has substantially the same transverse cross sectional shape as the penetration path in the eclipse shape of the scope/treatment instrument channel 44.

With the above-mentioned configuration, turning of the image catheter 2 and various treatment instruments in the scope/treatment instrument channel 44 about the longitudinal axis is restricted, and the image catheter 2 and various treatment instruments are stable and twisting is prevented. In the image catheter 2, the visual recognition direction can be easily identified, and the various treatment instruments have the improved operability.

It should be noted that as shown in FIG. 51, by respectively providing index portions 87a, 42d, 47, and 14 for regulating the inserting direction to an opening portion 87 on an operation portion side arranged in the operation portion 73 of the lateral vision endoscope 70, the distal end part of the guide catheter insertion portion 42 of the guide catheter 40, a proximal end part of the treatment instrument penetration portion 43 the guide catheter 40, and a distal end part of the scope insertion portion 12 of the image catheter 2, it is possible to regulate the inserting directions about the long axis for the respective corresponding insertions.

Furthermore, as shown in FIG. 52, the guide catheter 40 may be formed with a groove portion 56 to the proximal end along the guide portion 41 so that the groove portion 56 configuring a rail-shaped guide portion is in communication with the treatment instrument channel 44 of the guide catheter insertion portion 42. As a result, as shown in FIG. 53, by providing an engagement portion substantially matching to the shape of the groove portion 56 to the predetermined treatment instrument 59 inserted to the treatment instrument channel 44 by a whole length or at a distal end part, it is possible to regulate the inserting directions of the treatment instrument 59. Also, as the treatment instrument 59 is regularly engaged and held with an engaged portion 58 along the extending direction of the guide portion 41, the introduction property into the bile duct 52 or the pancreatic duct 53 is improved.

### (Ninth Embodiment)

Next, a ninth embodiment useful for understanding but not part of the invention will be described. According to the present embodiment, the guide portion 41 of the guide catheter 40, to which a characteristic configuration is added, will be described hereinafter.

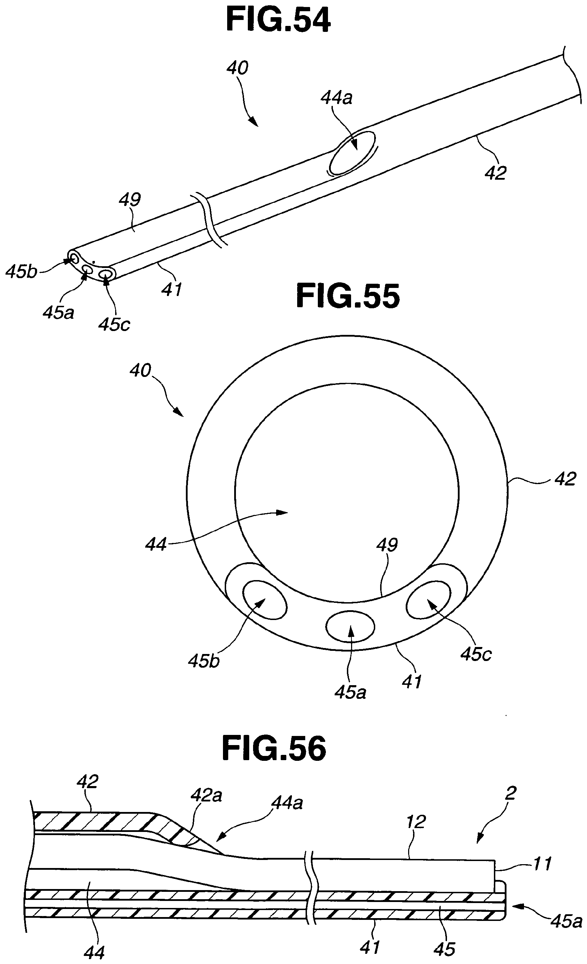

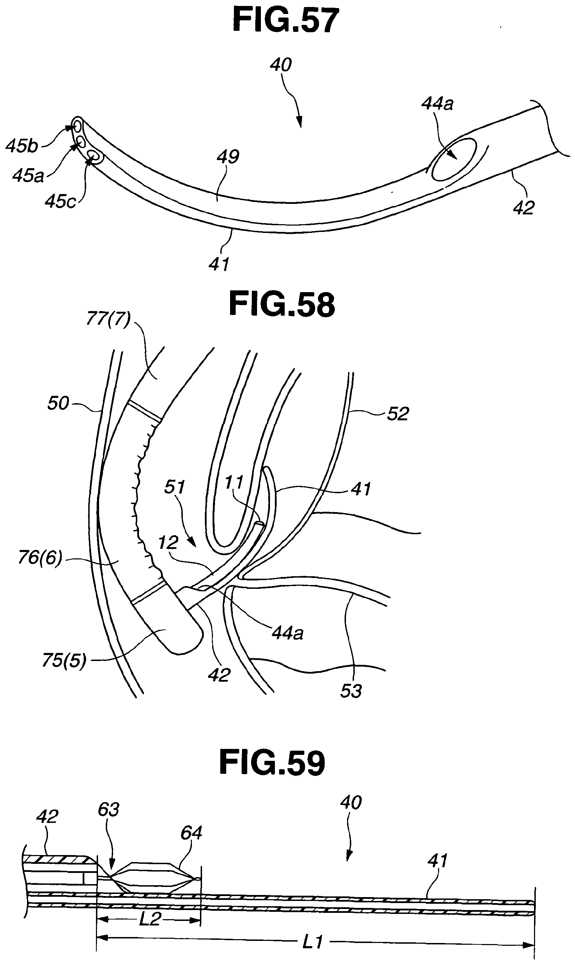

FIGS. 54 to 58 represent the ninth embodiment: FIG. 54 shows a configuration of a distal end part of the guide catheter, FIG. 55 shows the guide catheter as seen from its distal end part, FIG. 56 is a cross sectional view of a distal end part of a guide catheter which is a first modification example, FIG. 57 shows a configuration of a distal end part of a guide catheter which is a second modification example, and FIG. 58 shows a state in which the guide catheter which is the second modification example is inserted from a duodenal papilla into a bile duct. It should be noted that in the description on the present embodiment as well, the same reference numerals are used for the configurations described in the above embodiments and the detail description will be omitted.

As shown in FIGS. 54 and 55, the guide catheter 40 according to the present embodiment has a shape in which the transverse cross sectional shape of the guide portion 41 is bent along the outer peripheral portion. That is, the transverse cross sectional shape of the guide portion 41 is concave on the center axis side of the substantially circular guide catheter 40, and the concave surface has a concave, so-called spatular guide surface 49.

With such a configuration, in the guide catheter 40, the image catheter 2 or various treatment instruments introduced from the opening portion 44a at the distal end of the guide catheter insertion portion 42 is guided in a straight manner by the guide surface 49 of the guide portion 41 functioning as, so to speck, a rail. That is, the operator can easily insert the image catheter 2 or various treatment instruments along the guide surface 49 of the guide portion 41, from the papilla portion 51 to the bile duct 52 or the pancreatic duct 53.

Moreover, similarly to the embodiments as described above, other than the opening portion 45a of the fluid supply channel 45, two opening portions 45b and 45c are arranged on the distal end surface of the guide portion 41. The opening portions 45b and 45c are openings of the fluid supply path not shown that is different from the fluid supply path 43a extended to, the guide portion 41, the guide catheter insertion portion 42, and the treatment instrument penetration portion 43.

In other words, the guide catheter 40 includes three fluid supply paths (43a). As a result, with use of the three fluid supply paths (43a), the guide catheter 40 can selectively and simultaneously perform injection of the contrast agent as well as watering and airing, the suction, and the like for example. In particular, the suction allows removal of a living body fluid such as bile in the bile duct 52 or the pancreatic duct 53 or a gaseous matter such as air or gas. As a result, the operator can fill the contrast agent in the bile duct 52 or the pancreatic duct 53 sufficiently, whereby the abnormal area, the gallstone, and the like can be judged easily with the X-ray image.

It should be noted that as shown in FIG. 56, the guide catheter insertion portion 42 may have a protrusion portion 42a at the distal end part on the opposite side to the side where the guide portion 41 is extended, so as to push the image catheter 2 or various treatment instruments to the guide portion 41 side. As a result, the image catheter 2 or various treatment instruments is introduced from the opening portion 44a of the guide catheter insertion portion 42 and is then pressed against the guide surface 49 of the guide portion 41. For that reason, the operator can move the image catheter 2 or various treatment instruments along the guide surface 49 back and forward.

Furthermore, as shown in FIG. 57, the guide portion 41 may have a folding habit bending on the guide surface 49 side towards the extension side of the guide catheter insertion portion 42. As a result, as shown in FIG. 58, after being introduced from the opening portion 44a of the guide catheter insertion portion 42, the image catheter 2 or various treatment instruments follows the bending guide surface 49 of the guide portion 41 inserted into the bile duct 52. Therefore, even with such a configuration, the image catheter 2 or various treatment instruments easily moves beck and forwards along the guide surface 49.

### (Tenth Embodiment)

Next, a tenth embodiment useful for understanding but not part of the invention will be described. According to the present embodiment, a relation between the guide portion 41 of the guide catheter 40 according to the above-mentioned embodiments and various treatment instruments will be described.

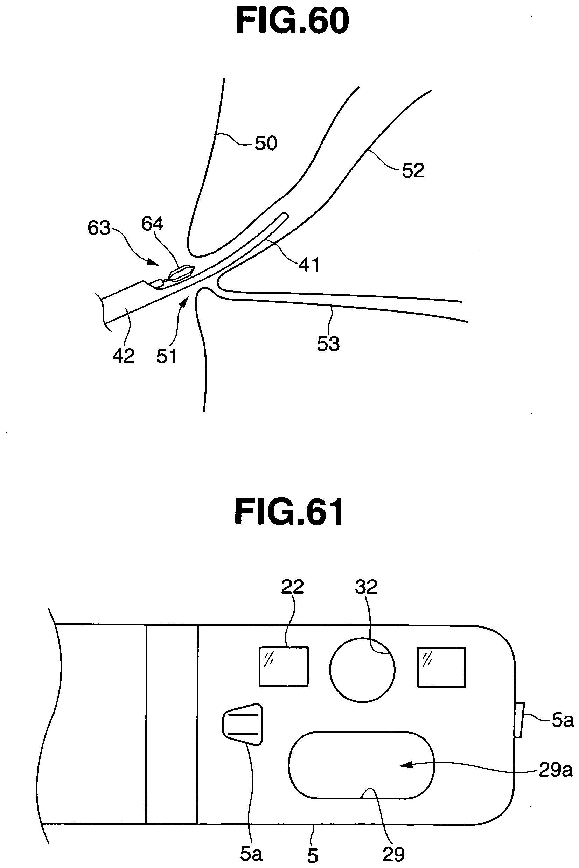

FIGS. 59 and 60 represent the tenth embodiment: FIG. 59 is a cross sectional view of a distal end part of a guide catheter to which basket grasping forceps are inserted and FIG. 60 shows a state in which a guide portion of the guide catheter to which the basket grasping forceps are inserted is inserted to the bile duct. It should be noted that in the description on the present embodiment as well, the same reference numerals are used for the configurations described in the above embodiments and the detail description will be omitted.

As shown in FIG. 59, the guide portion 41 of the guide catheter 40 according to the above-mentioned embodiments is longer than, herein, the basket wire 64 of the basket grasping forceps 63 functioning as the treatment instrument, in the longitudinal axis.

To be more specific, the axis length on the longitudinal direction of the guide portion 41 is set as L1, the length of the longitudinal axis direction of the basket wire 64 functioning as the treatment instrument of the basket grasping forceps 63 is set as L2. The length L2 of the guide portion 41 is set to be sufficiently longer than the basket wire length L2 (L1>L2).

In other words, as shown in FIG. 60, for example, even when the entire guide portion 41 inserted to the bile duct 52 from the papilla portion 51 is not removed from the bile duct 52, the entirety of the basket wire 64 of the basket grasping forceps 63 introduced from the guide catheter insertion portion 42 is located within the duodenum 50.

As a result, the operator can discharge the gallstone 54 picked up by the basket wire 64 to the duodenum 50, for example, while the guide portion 41 of the guide catheter 40 is inserted to the bile duct 52. In other words, the operator does not need to perform the insertion and removal operation on the guide portion 41 of the guide catheter 40 with respect to the bile duct 52 for operating the basket grasping forceps 63. It should be noted that in the present embodiment, the basket grasping forceps 63 has been described, but the length of the guide portion 41 L1 is set sufficiently longer than the length in the longitudinal axis direction of various treatment instruments, for example, such as biopsy forceps and a high frequency snare.

Therefore, in particular, once the operator inserts the guide portion 41 of the guide catheter 40 from the papilla portion 51 to the bile duct 52 or the pancreatic duct 53, until the treatment using the plural treatment instruments inserted or removed with respect to the guide catheter 40 is completed, re-approach to the bile duct 52 or the pancreatic duct 53 from the papilla portion 51 is not needed.

### (Eleventh Embodiment)

Next, an eleventh embodiment useful for understanding but not part of the invention will be described. According to the present embodiment, in addition to the penetration hole 30 of the endoscope 1 through which the image catheter 2 according to the second embodiment is penetrated, the guide catheter penetration hole portion through which the guide catheter 40 is freely penetrated is also added.

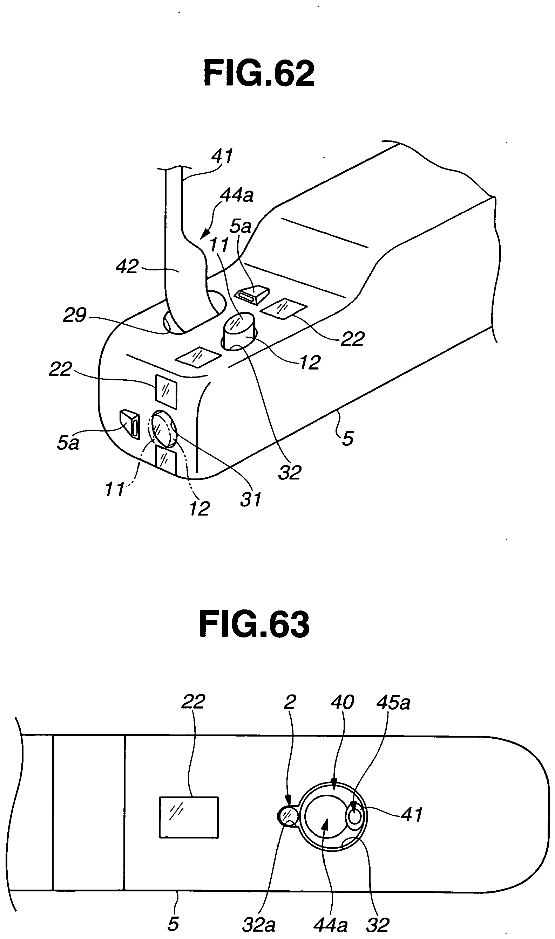

FIGS. 61 and 62 represent the eleventh embodiment: FIG. 61 is a plan view showing one side of a distal end portion of an endoscope and FIG. 62 is a perspective view showing the distal end portion of the endoscope. It should be noted that in the description on the present embodiment, the same reference numerals are used for the configurations described in the above embodiments and the detail description will be omitted.

As shown in FIGS. 61 and 62, the endoscope 1 according to the present embodiment includes a guide catheter penetration path 29 having an opening portion 29a on one side surface, in which the lateral vision hole 32 of the distal end portion 5 is opened.

The guide catheter penetration path 29 has the hole diameter of about 6 mm while being arranged from the insertion portion 3 to the operation portion 4, and although not shown in the drawing, is opened at the guide catheter insertion portion provided at the operation portion 4. That is, the guide catheter 40 is inserted to the guide catheter penetration path 29 and protrudes from the opening portion 29a of the distal end portion 5.

According to such a configuration of the endoscope 1, the operator can separately insert the image catheter 2 and the guide catheter 40 to the insertion portion 3 of the endoscope 1, whereby it is possible to use the guide catheter 40 for the insertion of the various treatment instruments alone.

Furthermore, as the operator inserts the image catheter 2 to the lateral vision hole 32 side, the state in which the various treatment instruments are inserted to the guide catheter 40 and the operation on the various treatment instruments can also be visually checked without performing the X-ray photography. It should be noted that at this time, the illumination light from the optical members 22 penetrates body tissues around the papilla portion 51, so that even the inside of the bile duct 52 or the pancreatic duct 53 can be visually checked.

### (Twelfth Embodiment)

Next, a twelfth embodiment useful for understanding but not part of the invention will be described. According to the present embodiment, the penetration hole 30 of the distal end portion 5 of the endoscope 1 according to the second embodiment, to which a characteristic configuration is added, will be described hereinafter.

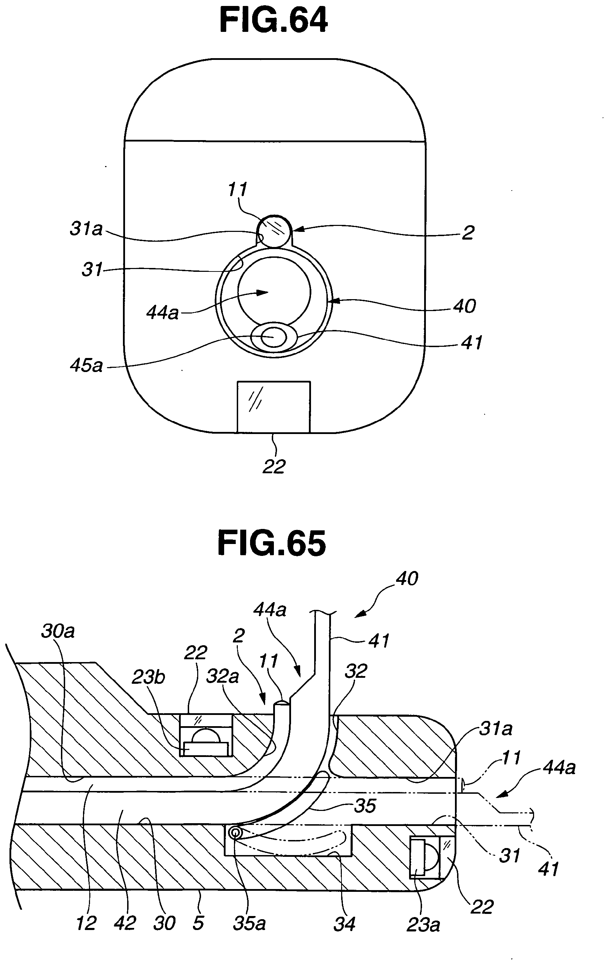

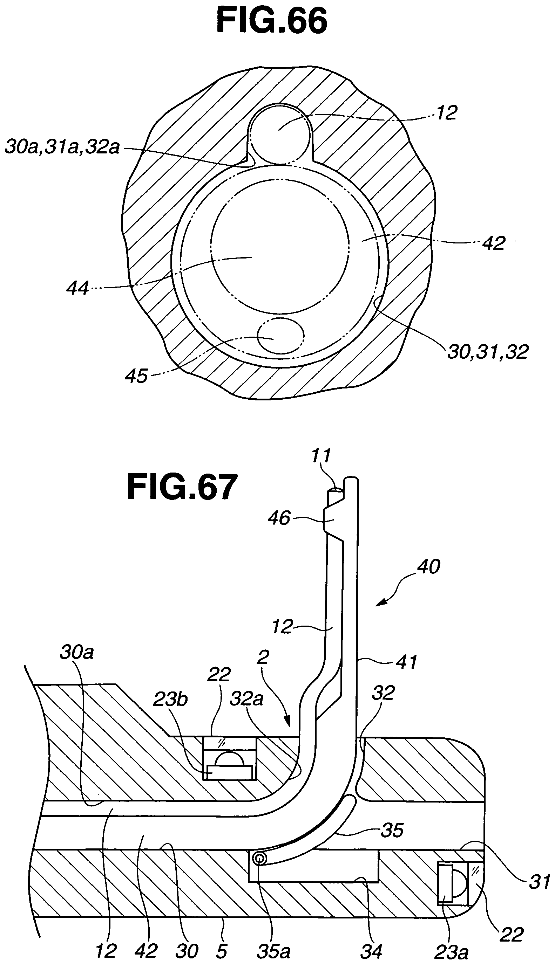

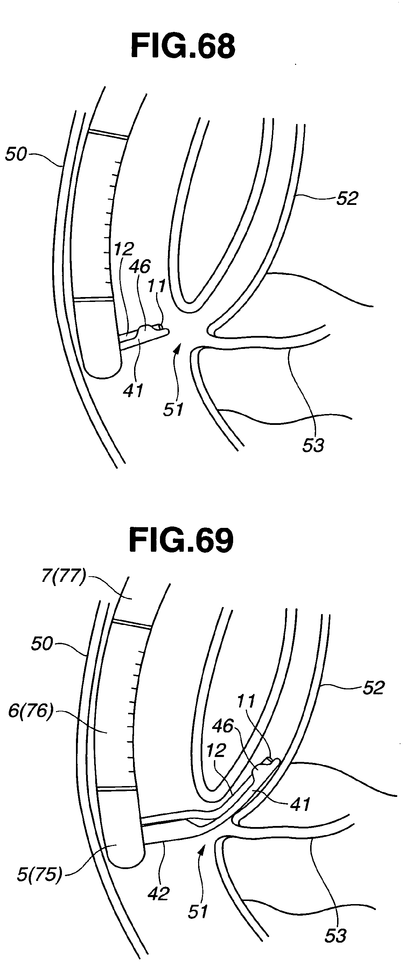

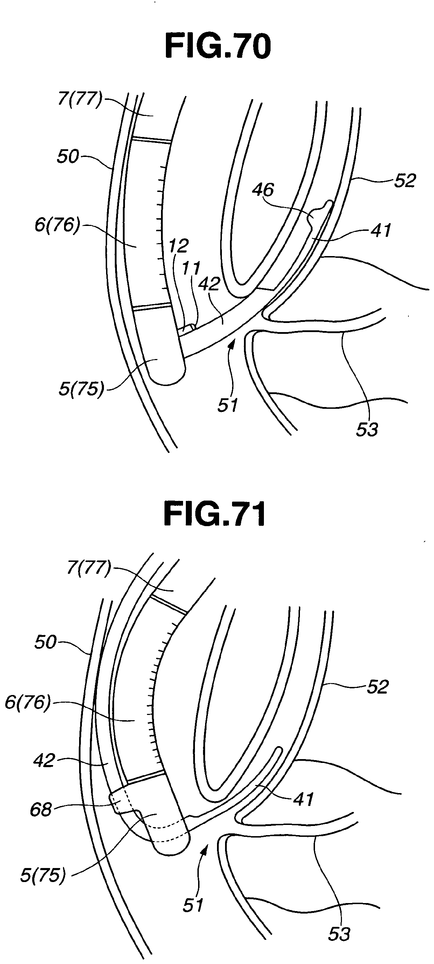

FIGS. 63 to 71 represent the twelfth embodiment: FIG. 63 is a plan view showing one side of a distal end portion of an endoscope, FIG. 64 is a plan view showing a distal end surface of the distal end portion of the endoscope, FIG. 65 is a cross sectional view of a distal end portion of the endoscope, FIG. 66 shows a hole portion inside the distal end portion, a straight vision hole portion, or a lateral vision hole portion, being a partial cross sectional view of the distal end portion which shows a scope penetration concave portion, FIG. 67 is a cross sectional view of the distal end portion of the endoscope in a case where the guide catheter provided with a holding portion to guide portion and the image catheter are used, FIG. 68 illustrates a state in which the distal end portion of the endoscope based on a configuration corresponding to FIG. 67 is inserted to a location in the vicinity of the papilla portion and the image catheter held by the holding portion of the guide portion slightly protrudes, FIG. 69 illustrates a state in which the guide portion is inserted into the bile duct together with the image catheter, FIG. 70 illustrates a state in which a distal end portion of a guide catheter insertion portion of the guide catheter is inserted into the bile duct and the image catheter is removed from the holding portion to pick up an image of the papilla portion, and FIG. 71 is an explanatory diagram for explaining an configuration in which the distal end portion of the endoscope is provided with a holding portion, representing a modification example. It should be noted that in the description on the present embodiment as well, the same reference numerals are used for the configurations described in the above embodiments and the detail description will be omitted.

As shown in FIGS. 63 to 66, outer peripheral portions of the penetration hole 30, the straight vision hole 31, and the lateral vision hole 32 arranged at the distal end portion 5 are respectively provided with image catheter penetration grooves 30a, 31a, and 32a through which the image catheter 2 is freely penetrated along the outer peripheral surface of the guide catheter 40.

The two image catheter penetration grooves 30a and 31a are formed with the same cross sectional shape along the same axis of the outer peripheral portion of the respective penetration holes 30 and 31 on one side surface of the distal end portion 5 on which the lateral vision hole 32 is opened. In addition, the image catheter penetration groove 32a is formed along the outer peripheral portion of the lateral vision hole 32 on the proximal end side of the distal end portion 5 with the same cross sectional shape as that of the respective grooves 30a and 31a.

The image catheter penetration groove 31a is formed up to the opening portion of the straight vision hole 31 at the distal end surface of the distal end portion 5. On the other hand, the image catheter penetration groove 32a is formed up to the opening portion of the lateral vision hole 32 on one side surface of the distal end portion 5.

The image catheter penetration grooves 30a to 32a are grooves set to make a clearance so as to hold the outer peripheral portion of the image catheter 2 in the state in which the image catheter 2 is penetrated.

It should be noted that in the endoscope channel 17 arranged from the proximal end part of the distal end portion 5 to the operation portion 4 as well, it is preferable to provide a groove portion having the same shape as the image catheter penetration grooves 30a to 32a, and to which the image catheter 2 is freely penetrated in the same direction as the image catheter penetration groove 30a.

Therefore, in the endoscope 1 according to the present embodiment, without being penetrated through the guide catheter 40, the image catheter 2 can be penetrated to the insertion portion 3 along the outer peripheral portion of the guide catheter 40, whereby the view direction can be set to face the direction in which the distal end part of the guide catheter 40 faces. With the configuration of the endoscope 1, the above-mentioned effects as those of the tenth embodiment can be achieved.

Next, a case in which the lateral/straight freely changeable endoscope 1 according to the present embodiment and the guide catheter 40 provided to the holding portion 46 arranged in the guide portion 41 and the image catheter 2 which are described in the third embodiment are used will be described by using FIGS. 67 to 70.

As shown in FIG. 67, in the guide catheter 40 having the holding portion 46 in the guide portion 41, the guide catheter 40 can be penetrated through the penetration hole 30 of the endoscope 1, and also the image catheter 2 whose distal end is held by the holding portion 46 of the guide portion 41 can be inserted into the image catheter penetration grooves 30a at the same time.

As shown in FIG. 68, the operator can confirm the position of the papilla portion 51 of the duodenum 50 with the image catheter 2 held by the guide catheter 40, and then, by way of the video of the image catheter 2, while visually checking an approach to the papilla portion 51, as shown in FIG. 69, the guide portion 41 of the guide catheter 40 can be reliably inserted into, herein, the bile duct 52.

Then, the operator pulls only the image catheter 2 to the hand side to remove the distal end part of the image catheter 2 from the holding portion 46 at the guide portion 41 of the guide catheter 40 via the papilla portion 51, and as shown in FIG. 70, the distal end surface 11 of the image catheter 2 is set to be located within the duodenum 50 so as to slightly protrude from the distal end portion 5 of the endoscope 1. As a result, the operator can visually check the state of the papilla portion 51 by way of the video of the image catheter 2. That is, the operator can use the image catheter 2 as an image pickup apparatus of the endoscope 1. It should be noted that herein, the endoscope 1 has been exemplified, but of course, the use for the lateral vision endoscope 70 described in the first embodiment is also similarly possible.

In addition, as a modified example of the holding portion 46 described in the third embodiment and the present embodiment, as shown in FIG. 71, the endoscope 1, or the distal end portions 5 and 75 of the lateral vision endoscope 70 may be provided with a holding portion 68 for penetrating and holding the guide catheter insertion portion 42 of the guide catheter 40. At this time, penetration holes for holding the guide catheter 40 from the side where the holding portion 68 is provided to the opposite side and also allowing a protrusion are formed in the distal end portions 5 and 75.

In the technologies described in the embodiments, the invention described in the embodiments is not limited to the respective embodiments, and can have various modifications without departing from the gist of the invention in the practice stage. Furthermore, the embodiments described above contain the inventions in various stages and various inventions can be extracted in appropriate combinations of the plural disclosed components.

For example, when some of the components from the whole components shown in the respective embodiments are deleted, if the effect described in the portion of Effect of the Invention can be obtained, a configuration with this component deleted can be extracted as the invention.

It should be noted that an embodiment may be provided with features or a combination of features as described in the following:
A guiding elongated medical membermay include:
a proximal end area having a predetermined length and outer diameter with a substantially circular cross sectional shape;
a distal end area which is provided so as to extend from a distal end of the proximal end area and has an outer diameter smaller than the proximal end area;
a first lumen which is arranged in the proximal end area, opened at a near end and a remote end of the distal end area, and also adapted to allow a penetration of at least an operation medical member for carrying out a predetermined medical action; and
at least one first hole portion which is in communication with the first lumen and is arranged in a side peripheral portion of the proximal end area.

A guiding elongated medical member may include:
a proximal end area having a predetermined length and outer diameter with a substantially circular cross sectional shape;
a distal end area which is provided so as to extend from a distal end of the proximal end area and has an outer diameter smaller than the proximal end area; and
a first lumen which is arranged in the proximal end area, opened at a near end and a remote end of the distal end area, and also adapted to allow a penetration of at least an operation medical member for carrying out a predetermined medical action,
wherein the distal end area includes a guide portion which is provided so as to extend from a position in the vicinity of a margin of the near end surface of the proximal end area and adapted to guide the operation medical member extending from the opening on the near end side in an extending direction.

The guiding elongated medical member may further include:
a second lumen which is arranged in the distal end area and opened at a distal end and on the remote end side of the proximal end area; and
at least one second hole portion which is arranged in the proximal end area and is in communication with the second lumen.

The guiding elongated medical member may, further include: a first hole portion which is arranged in the proximal end area and is in communication with the first lumen.

The proximal end area may have a slit formed from the opening at the near end to the remote end with a predetermined length.

The guiding elongated medical member may further include a second lumen which is arranged in the distal end area and opened at the distal end and on the remote end side of the proximal end area.

The guiding elongated medical member may further include a holding portion which is arranged at a distal end part of the distal end area, for penetration-holding the operation medical member.

The first lumen may have substantially a same shape as an external shape of the operation medical member to be engaged therewith for preventing a rotation of the operation medical member.

The remote end part of the proximal end area may be provided with an index portion for inserting the operation medical member in a predetermined direction in accordance with an instruction display of an operation direction of the operation medical member.

The guiding elongated medical member distal end area may be longer than a length of a treatment portion of the operation medical member.

An elongated medical apparatus may include:
an image pickup apparatus including:
   an introduction portion which has a long length and is provided with an observation light transfer member; and
   an image pickup portion adapted to pick up an observation light which is introduced to the observation light transfer member; and
a guiding elongated medical member including:
   a proximal end area having a predetermined length and outer diameter with a substantially circular cross sectional shape;
   a distal end area which is provided so as to extend from a distal end of the proximal end area and has an outer diameter smaller than the proximal end area;
   a first lumen which is arranged in the proximal end area, opened at a near end and a remote end of the distal end area, and also adapted to allow a penetration of the introduction portion of the image pickup apparatus; and
   at least one first hole portion which is in communication with the first lumen and is arranged in a side peripheral portion of the proximal end area.

An elongated medical apparatus may include:
an image pickup apparatus including:
   an introduction portion which has a long length and is provided with an observation light transfer member; and
   an image pickup portion adapted to pick up an observation light which is introduced to the observation light transfer member; and
a guiding elongated medical member including:
   a proximal end area having a predetermined length and outer diameter with a substantially circular cross sectional shape;
   a distal end area which is provided so as to extend from a distal end of the proximal end area and has an outer diameter smaller than the proximal end area; and
   a first lumen which is arranged in the proximal end area, opened at a near end and a remote end of the distal end area, and also adapted to allow a penetration of the introduction portion of the image pickup apparatus for carrying out a predetermined medical action,
wherein the distal end area includes a guide portion which is provided so as to extend from a position in the vicinity of a margin of the near end surface of the proximal end area and adapted to guide the operation medical member extending from the opening on the near end side in an extending direction.

The elongated medical apparatus may further include a holding portion which is arranged at a distal end part of the distal end area, for penetration-holding the introduction portion of the image pickup apparatus.

The first lumen may have substantially a same shape as an external shape of the introduction portion to be engaged therewith for preventing a rotation of the introduction portion of the image pickup apparatus.

The remote end part of the proximal end area may be provided with an index portion for inserting the introduction portion in a predetermined direction in accordance with an instruction display of an operation direction of the introduction portion of the image pickup apparatus.

An elongated medical apparatus may include:
an elongated medical member including:
   an insertion portion which is inserted into a body cavity and provided with a channel through which a medical member can be freely penetrated; and
   a varying mechanism portion which is arranged at a distal end portion of the insertion portion and adapted to switch a facing direction of the medical member penetrated through the channel; and
a guiding elongated medical member including:
   a proximal end area having a predetermined length and outer diameter with a substantially circular cross sectional shape;
   a distal end area which is provided so as to extend from a distal end of the proximal end area and has an outer diameter smaller than the proximal end area;
   a first lumen which is arranged in the proximal end area, opened at a near end and a remote end of the distal end area, and also adapted to allow a penetration of at least an operation medical member for carrying out a predetermined medical action; and
   at least one first hole portion which is in communication with the first lumen and is arranged in a side peripheral portion of the proximal end area.

An elongated medical apparatus may include:
an elongated medical member including:
   an insertion portion which is inserted into a body cavity and provided with a channel through which a medical member can be freely penetrated; and
   a varying mechanism portion which is arranged at a distal end portion of the insertion portion and adapted to switch a facing direction of the medical member penetrated through the channel; and
a guiding elongated medical member including:
   a proximal end area having a predetermined length and outer diameter with a substantially circular cross sectional shape;
   a distal end area which is provided so as to extend from a distal end of the proximal end area and has an outer diameter smaller than the proximal end area; and
   a first lumen which is arranged in the proximal end area, opened at a near end and a remote end of the distal end area, and also adapted to allow a penetration of at least an operation medical member for carrying out a predetermined medical action,
wherein the distal end area includes a guide portion which is provided so as to extend from a position in the vicinity of a margin of the near end surface of the proximal end area and adapted to guide the operation medical member extending from the opening on the near end side in an extending direction.

An elongated medical apparatus may include:
an elongated medical member including:
   an insertion portion which is inserted into a body cavity and provided with a channel through which a medical member can be freely penetrated; and
   a varying mechanism portion which is arranged at a distal end portion of the insertion portion and adapted to switch a facing direction of the medical member penetrated through the channel;
an image pickup apparatus including:
   an introduction portion which has a long length and is provided with an observation light transfer member; and
   an image pickup portion adapted to pick up an observation light which is introduced to the observation light transfer member; and
a guiding elongated medical member including:
   a proximal end area having a predetermined length and outer diameter with a substantially circular cross sectional shape;
   a distal end area which is provided so as to extend from a distal end of the proximal end area and has an outer diameter smaller than the proximal end area;
   a first lumen which is arranged in the proximal end area, opened at a near end and a remote end of the distal end area, and also adapted to allow a penetration of the introduction portion of the image pickup apparatus or an operation medical member for carrying out a predetermined medical action; and
   at least one first hole portion which is in communication with the first lumen and is arranged in a side peripheral portion of the proximal end area.

An elongated medical apparatus may include:
an elongated medical member including:
   an insertion portion which is inserted into a body cavity and provided with a channel through which a medical member can be freely penetrated; and
   a varying mechanism portion which is arranged at a distal end portion of the insertion portion and adapted to switch a facing direction of the medical member penetrated through the channel;
an image pickup apparatus including:
   an introduction portion which has a long length and is provided with an observation light transfer member; and
   an image pickup portion adapted to pick up an observation light which is introduced to the observation light transfer member; and
a guiding elongated medical member including:
   a proximal end area having a predetermined length and outer diameter with a substantially circular cross sectional shape;
   a distal end area which is provided so as to extend from a distal end of the proximal end area and has an outer diameter smaller than the proximal end area; and
   a first lumen which is arranged in the proximal end area, opened at a near end and a remote end of the distal end area, and also adapted to allow a penetration of the introduction portion of the image pickup apparatus or an operation medical member for carrying out a predetermined medical action,
wherein the distal end area includes a guide portion which is provided so as to extend from a position in the vicinity of a margin of the near end surface of the proximal end area and adapted to guide the operation medical member extending from the opening on the near end side in an extending direction.

The channel of the elongated medical member may have a groove portion through which the introduction portion of the image pickup apparatus can be penetrated in parallel with the guiding elongated medical member or the operation medical member.

An elongated medical apparatus may include:
an elongated medical member including:
   an insertion portion which is inserted into a body cavity and provided with a channel through which a medical member can be freely penetrated; and
   a varying mechanism portion which is arranged at a distal end portion of the insertion portion and adapted to switch a facing direction of the medical member penetrated through the channel; and
an image pickup apparatus including:
   a long introduction portion which is provided with an observation light transfer member and can be freely inserted into the channel of the elongated medical member; and
   an image pickup portion adapted to pick up an observation light which is introduced to the observation light transfer member.

A procedure method of using a guiding elongated medical member may include a proximal end area having a predetermined length and outer diameter with a substantially circular cross sectional shape; a distal end area which is provided so as to extend from a distal end of the proximal end area and has an outer diameter smaller than the proximal end area; and a first lumen which is arranged in the proximal end area, opened at a near end and a remote end of the distal end area, and also adapted to allow penetrations of at least first and second operation medical member for carrying out a predetermined medical action in a bile path/pancreatic area inside a body cavity of a living body, the method comprising:
via an elongated medical member having an insertion portion which can be inserted into the body cavity of the living body, arranging the guiding elongated medical member at a position in a vicinity of the bile path/pancreatic area in a duodenal area,
allowing a distal end of the guiding elongated medical member to extend from the insertion portion of the elongated medical member to insert the distal end area of the guiding medical member from the duodenal area into the bile path/pancreatic area.

The procedure method of using the guiding elongated medical member, wherein the first operation treatment instrument is an image pickup apparatus including an introduction portion which has a long length, is provided with an observation light transfer member, and can be freely penetrated into the channel of the elongated medical member and an image pickup portion adapted to pick up an observation light which is introduced to the observation light transfer member, may further comprise:
inserting the introduction portion into the first lumen from an opening on the remote end side before inserting or after inserting the guiding elongated medical member into the insertion portion;
arranging a distal end part of the introduction portion in an opening on the near end side; and
inserting a distal end part of the introduction portion from the duodenal area into the bile path/pancreatic area while observing the bile path/pancreatic area by way of the image pickup apparatus.

The procedure method of using the guiding elongated medical member may further comprise:
inserting the first or second operation medical member into the first lumen from an opening on the remote end side;
inserting a first or second treatment portion arranged at a distal end portion of the first or second operation medical member from an opening on the near end side into the bile path/pancreatic area; and
executing a predetermined medical action with the first or second treatment portion.

The procedure method of using the guiding elongated medical member, wherein the first or second operation treatment instrument is a tube body, may further comprise:
inserting the tube body into the first lumen from the opening on the remote end side;
inserting a distal end part of the tube body into the bile path/pancreatic area from the opening on the near end side; and
injecting a contrast agent via the tube body into the bile path/pancreatic area.

The procedure method of using the guiding elongated medical member, wherein the first or second operation treatment instrument is a high frequency treatment instrument provided with a high frequency knife in the first or second treatment portion, may further comprise:
inserting the high frequency treatment instrument into the first lumen from the opening on the remote end side;
inserting the high frequency knife into the bile path/pancreatic area from the opening on the opening on the near end side; and
cutting a part of the bile path/pancreatic area.

The procedure method of using the guiding elongated medical member, wherein the first or second operation treatment instrument is a collection treatment instrument provided with a collection portion in the first or second treatment portion, may further comprise:
inserting the collection treatment instrument into the first lumen from the opening on the remote end side;
inserting the collection portion into the bile path/pancreatic area from the opening on the near end side; and
collecting a foreign matter within the bile path/pancreatic area.

The procedure method of using the guiding elongated medical member, may further comprise:
removing the introduction portion of the image pickup apparatus from the first lumen;
inserting the second operation medical member into the first lumen from the opening on the remote end side;
inserting the second treatment portion arranged in the distal end part of the second operation medical member into the bile path/pancreatic area from the opening on the near end side; and
executing a predetermined medical action with the second treatment portion.

The procedure method of using the guiding elongated medical member, wherein the second operation treatment instrument is a tube body, may further comprise:
inserting the tube body into the first lumen from the opening on the remote end side;
inserting a distal end part of the tube body into the bile path/pancreatic area from the opening on the near end side; and
injecting a contrast agent via the tube body into the bile path/pancreatic area.

The procedure method of using the guiding elongated medical member, wherein the second operation treatment instrument is a high frequency treatment instrument provided with a high frequency knife in the second treatment portion, may further comprise:
inserting the high frequency treatment instrument into the first lumen from the opening on the remote end side;
inserting the high frequency knife into the bile path/pancreatic area from the opening on the near end side; and
cutting a part of the bile path/pancreatic area.

The procedure method of using the guiding elongated medical member, wherein the second operation treatment instrument is a collection treatment instrument provided with a collection portion in the second treatment portion, may further comprise:
inserting the collection treatment instrument into the first lumen from the opening on the remote end side;
inserting the collection portion into the bile path/pancreatic area from the opening on the near end side; and
collecting a foreign matter within the bile path/pancreatic area.

The procedure method of using the guiding elongated medical member, may further comprise:
removing, after a predetermined medical action is performed, the second operation medical member from the first lumen;
further using a third operation medical member which is penetrated through the first lumen and provided at a distal end thereof with a third treatment portion for performing a predetermined medical action in the bile path/pancreatic area inside the body cavity of the living body;
inserting the third treatment portion arranged at the distal end part of the third operation medical member into the bile path/pancreatic area from the opening on the near end side; and
executing a predetermined medical action with the third treatment portion.

The procedure method of using the guiding elongated medical member, may further comprise:
inserting the near end part having an opening of the proximal end area into the bile path/pancreatic area.

The procedure method of using the guiding elongated medical member, wherein the first operation treatment instrument is an image pickup apparatus including: a long introduction portion which is provided with an observation light transfer member and can be freely inserted into the channel of the elongated medical member; and an image pickup portion adapted to pick up an observation light which is introduced to the observation light transfer member may further comprise:
inserting the introduction portion into the first lumen from the opening on the remote end side before inserting or after inserting the guiding elongated medical member into the insertion portion; and
inserting the distal part of the introduction portion from the duodenal area into the bile path/pancreatic area

The procedure method of using the guiding elongated medical member, wherein the first or second operation treatment instrument is a tube body, may further comprise:
inserting the tube body into the first lumen from the opening on the remote end side;
inserting a distal end part of the tube body into the bile path/pancreatic area from the opening on the near end side; and
injecting a contrast agent via the tube body into the bile path/pancreatic area.

The procedure method of using the guiding elongated medical member, wherein the first or second operation treatment instrument is a high frequency treatment instrument provided with a high frequency knife in the first or second treatment portion, may further comprise:
inserting the high frequency treatment instrument into the first lumen from the opening on the remote end side;
inserting the high frequency knife into the bile path/pancreatic area from the opening on the near end side; and
cutting a part of the bile path/pancreatic area.

The procedure method of using the guiding elongated medical member, wherein the first or second operation treatment instrument is a collection treatment instrument provided with a collection portion in the first or second treatment portion, may further comprise:
inserting the collection treatment instrument into the first lumen from the opening on the remote end side;
inserting the collection portion into the bile path/pancreatic area from the opening on the near end side; and
collecting a foreign matter within the bile path/pancreatic area.

The procedure method of using the guiding elongated medical member, may further comprise:
further removing the introduction portion of the image pickup apparatus from the first lumen;
inserting the second operation medical member into the first lumen from the opening on the remote end side;
inserting the second treatment portion arranged in the distal end part of the second operation medical member into the bile path/pancreatic area from the opening on the near end side; and
executing a predetermined medical action with the second treatment portion.

The procedure method of using the guiding elongated medical member, wherein the second operation treatment instrument is a tube body, may further comprise:
inserting the tube body into the first lumen from the opening on the remote end side;
inserting a distal end part of the tube body into the bile path/pancreatic area from the opening on the near end side; and
injecting a contrast agent via the tube body into the bile path/pancreatic area.

The procedure method of using the guiding elongated medical member, wherein the second operation treatment instrument is a high frequency treatment instrument provided with a high frequency knife in the second treatment portion may further comprise:
inserting the high frequency treatment instrument into the first lumen from the opening on the remote end side;
inserting the high frequency knife into the bile path/pancreatic area from the opening on the near end side; and
cutting a part of the bile path/pancreatic area.

The procedure method of using the guiding elongated medical member, wherein the second operation treatment instrument is a collection treatment instrument provided with a collection portion in the second treatment portion, may further comprise:
inserting the collection treatment instrument into the first lumen from the opening on the remote end side;
inserting the collection portion into the bile path/pancreatic area from the opening on the near end side; and
collecting a foreign matter within the bile path/pancreatic area.

The procedure method of using the guiding elongated medical member, may further comprise:
removing, after the predetermined medical action is executed, the second operation medical member from the first lumen;
further using a third operation medical member which is penetrated through the first lumen and provided at a distal end thereof with a third treatment portion for performing a predetermined medical action in the bile path/pancreatic area inside the body cavity of the living body;
inserting the third treatment portion arranged at the distal end part of the third operation medical member into the bile path/pancreatic area from the opening on the near end side; and
executing a predetermined medical action with the third treatment portion.

The procedure method of using the guiding elongated medical member, may further comprise:
injecting a contrast agent via the first lumen into the bile path/pancreatic area.

The procedure method of using the guiding elongated medical member, wherein the first or second operation treatment instrument is a high frequency treatment instrument provided with a high frequency knife in the first or second treatment portion, may further comprise:
inserting the high frequency treatment instrument into the first lumen from the opening on the remote end side;
inserting the high frequency knife into the bile path/pancreatic area from the opening on the near end side;
using the contrast agent to pick up an image of a state of the bile path/pancreatic area by way of X ray; and
cutting a part of the bile path/pancreatic area.

The procedure method of using the guiding elongated medical member, wherein the first or second operation treatment instrument is a collection treatment instrument provided with a collection portion in the first or second treatment portion, may further comprise:
inserting the collection treatment instrument into the first lumen from the opening on the remote end side;
inserting the collection portion into the bile path/pancreatic area from the opening on the near end side;
using the contrast agent to pick up an image of a state of the bile path/pancreatic area by way of X ray; and
collecting a foreign matter within the bile path/pancreatic area.

The procedure method of using the guiding elongated medical member, wherein the first operation treatment instrument is an image pickup apparatus including: a long introduction portion which is provided with an observation light transfer member and can be freely inserted into the channel of the elongated medical member; and an image pickup portion adapted to pick up an observation light which is introduced to the observation light transfer member, may further comprise:
inserting the introduction portion into the first lumen from the opening on the remote end side before inserting or after the guiding elongated medical member into the insertion portion; and
inserting the distal end part of the introduction portion from the duodenal area into the bile path/pancreatic area.

The procedure method of using the guiding elongated medical member, may further comprise:
further removing the introduction portion of the image pickup apparatus from the first lumen;
inserting the second operation medical member into the first lumen from the opening on the remote end side;
inserting the second treatment portion arranged in the distal end part of the second operation medical member into the bile path/pancreatic area from the opening on the near end side;
using the contrast agent to pick up an image of a state of the bile path/pancreatic area by way of X ray; and
executing a predetermined medical action with the second treatment portion.

The procedure method of using the guiding elongated medical member, wherein the second operation treatment instrument a high frequency treatment instrument provided with a high frequency knife in the second treatment portion, may further comprise:
inserting the high frequency treatment instrument into the first lumen from the opening on the remote end side;
inserting the high frequency knife into the bile path/pancreatic area from the opening on the near end side;
using the contrast agent to pick up an image of a state of the bile path/pancreatic area by way of X ray; and
cutting a part of the bile path/pancreatic area.

The procedure method of using the guiding elongated medical member, wherein the second operation treatment instrument is a collection treatment instrument provided with a collection portion in the second treatment portion, may further comprise:
inserting the collection treatment instrument into the first lumen from the opening on the remote end side;
inserting the collection portion into the bile path/pancreatic area from the opening on the near end side;
using the contrast agent to pick up an image of a state of the bile path/pancreatic area by way of X ray; and
collecting a foreign matter within the bile path/pancreatic area.

The procedure method of using the guiding elongated medical member, may further comprise:
removing, after the predetermined medical action is executed, the second operation medical member from the first lumen;
further using a third operation medical member which is penetrated through the first lumen and provided at a distal end thereof with a third treatment portion for performing a predetermined medical action in the bile path/pancreatic area inside the body cavity of the living body;
inserting the third treatment portion arranged at the distal end part of the third operation medical member into the bile path/pancreatic area from the opening on the near end side;
using the contrast agent to pick up an image of a state of the bile path/pancreatic area by way of X ray; and
executing a predetermined medical action with the third treatment portion.

The procedure method of using the guiding elongated medical member, wherein the distal end area is provided with a second lumen opened at a distal end and on the remote end side of the proximal end area, may further comprise:
inserting an introduction portion of an image pickup apparatus including the introduction portion which has a long length, is provided with an observation light transfer member, and an image pickup portion adapted to pick up an observation light which is introduced to the observation light transfer member into the second lumen before inserting or after inserting the guiding elongated medical member to the insertion portion from the opening on the remote end side;
inserting a distal end part of the introduction portion into the bile path/pancreatic area from an opening on the near end side; and
inserting the first operation medical member from the first lumen into the bile path/pancreatic area.

The procedure method of using the guiding elongated medical member, may further comprise:
further inserting the first or second operation medical member to the first lumen from the opening on the remote end side;
inserting a first or second treatment portion arranged at a distal end part of the first or second operation medical member from the opening on the near end side into the bile path/pancreatic area; and
executing a predetermined medical action with the first or second treatment portion.

The procedure method of using the guiding elongated medical member, wherein the first or second operation treatment instrument is a tube body, may further comprise:
inserting the tube body into the first lumen from the opening on the remote end side;
inserting a distal end part of the tube body into the bile path/pancreatic area from the opening on the near end side; and
injecting a contrast agent via the tube body into the bile path/pancreatic area.

The procedure method of using the guiding elongated medical member, wherein the first or second operation treatment instrument is a high frequency treatment instrument provided with a high frequency knife in the first or second treatment portion, may further comprise:
inserting the high frequency treatment instrument into the first lumen from the opening on the remote end side;
inserting the high frequency knife into the bile path/pancreatic area from the opening on the near end side; and
cutting a part of the bile path/pancreatic area.

The procedure method of using the guiding elongated medical member, wherein the first or second operation treatment instrument is a collection treatment instrument provided with a collection portion in the first or second treatment portion, may further comprise:
inserting the collection treatment instrument into the first lumen from the opening on the remote end side;
inserting the collection portion into the bile path/pancreatic area from the opening on the near end side; and
collecting a foreign matter within the bile path/pancreatic area.

The procedure method of using the guiding elongated medical member, may further comprise:
further removing the introduction portion of the image pickup apparatus from the second lumen;
inserting the first or second operation medical member into the second lumen from the opening on the remote end side;
inserting the first or second treatment portion arranged at the distal end part of the first or second operation medical member from the opening on the near end side into the bile path/pancreatic area; and
executing a predetermined medical action with the first or second treatment portion.

The procedure method of using the guiding elongated medical member, may further comprise:
inserting the near end part having an opening of the proximal end area into the bile path/pancreatic area.

The procedure method of using the guiding elongated medical member, wherein the first or second operation treatment instrument is a tube body, may further comprise:
inserting the tube body into the first lumen from the opening on the remote end side;
inserting a distal end part of the tube body into the bile path/pancreatic area from the opening on the near end side; and
injecting a contrast agent via the tube body into the bile path/pancreatic area.

The procedure method of using the guiding elongated medical member, wherein the first or second operation treatment instrument is a high frequency treatment instrument provided with a high frequency knife in the first or second treatment portion, may further comprise:
inserting the high frequency treatment instrument into the first lumen from the opening on the remote end side;
inserting the high frequency knife into the bile path/pancreatic area from the opening on the near end side; and
cutting a part of the bile path/pancreatic area.

The procedure method of using the guiding elongated medical member, wherein the first or second operation treatment instrument is a collection treatment instrument provided with a collection portion in the first or second treatment portion, may further comprise:
inserting the collection treatment instrument into the first lumen from the opening on the remote end side;
inserting the collection portion into the bile path/pancreatic area from the opening on the near end side; and
collecting a foreign matter within the bile path/pancreatic area.

The procedure method of using the guiding elongated medical member, may further comprise:
further removing the introduction portion of the image pickup apparatus from the second lumen;
inserting the first or second operation medical member to the second lumen from the opening on the remote end side;
inserting the first or second treatment portion arranged at the distal end part of the first or second operation medical member from the opening on the near end side into the bile path/pancreatic area; and
executing a predetermined medical action with the first or second treatment portion.

The procedure method of using the guiding elongated medical member, wherein:
the distal end area is provided with a penetration hole opened at a distal end and on the remote end side of the proximal end area; and
the first operation treatment instrument is an image pickup apparatus including an introduction portion which has a long length, is provided with an observation light transfer member, and can be freely penetrated into the channel of the elongated medical member and an image pickup portion adapted to pick up an observation light which is introduced to the observation light transfer member, the method further comprising:
   inserting the introduction portion from the opening on the remote end side into the penetration hole before inserting or after inserting the guiding elongated medical member into the insertion portion;
   arranging a distal end part of the introduction portion in the opening on the near end side of the penetration hole;
   further inserting the second operation medical member into the first lumen from the opening on the remote end side;
   inserting a second treatment portion arranged at a distal part of the second operation medical member from the opening on the near end side into the bile path/pancreatic area; and
   executing a predetermined medical action with the second treatment portion.

## Claims

1. A system comprising an elongated medical member (40) and an operation treatment instrument (61), the system comprising:
an insertion portion (42) having a long length, which is provided with a lumen (44) through which an operation treatment instrument (61) can be penetrated, the operation treatment instrument (61) being adapted to be formed into an arcuate shape by a distal end thereof being pulled, and including a wire cutter (62) for cutting a living body tissue, the lumen (44) being provided along a longitudinal axis of the insertion portion (42), wherein the insertion portion (42) is bendable and includes a distal end area including an opening portion (44a) of the lumen (44) at a distal end surface of the insertion portion (42) and a proximal end area having a predetermined length toward a proximal end side in an axis direction from the distal end area;
the elongated medical member further comprises a guide portion (41) adapted for guiding the operation treatment instrument (61) extended from the lumen (44) into a body cavity along the guide portion (41) together with the insertion portion (42), wherein a proximal end is connected to a peripheral portion of the opening portion (44a) of the lumen (44) and the guide portion (41) is provided to extend from the proximal end to a distal end toward a direction in which the opening portion (44a) is opened, in order to guide a distal end part of the operation treatment instrument (61) protruded from the opening portion (44a) of the lumen (44) to a target area, and
a slit (48a) having a predetermined length and formed from a distal end of a peripheral portion of the opening portion (44a) in a most proximal area of the opening portion (44a), the slit (48a) including at a proximal end part thereof a long hole (48b) and being adapted for allowing the wire cutter (62) to pass through from inside the lumen (44) without an entirety of the wire cutter (62) being guided out from the opening portion (44a).

2. The system including the elongated medical member (40) and the operation treatment instrument (61) according to claim 1, wherein the guide portion (41) can be elastically deformed.

3. The system including the elongated medical member (40) and the operation treatment instrument (61) according to claim 1 or 2, wherein an opening (45a) is formed on a distal end surface of the guide portion (41), and a fluid supply lumen (45) is formed in the insertion portion (42) and the guide portion (41).

4. The system including the elongated medical member (40) and the operation treatment instrument (61) according to any one of claims 1 to 3, wherein at least one hole portion (47a, 47b) which is in communication with the lumen (44) is formed on a distal end side outer peripheral portion of the insertion portion (42).

5. The system including the elongated medical member (40) and the operation treatment instrument (61) according to any one of claims 1 to 4, wherein a groove portion (56) which is in communication with the lumen (44) of the insertion portion (42) and is extended to the guide portion (41) is formed, the groove portion (56) having a shape substantially coincident with an engagement portion (58) provided to the operation treatment instrument.

6. The system including the elongated medical member (40) and the operation treatment instrument (61) according to any one of claims 1 to 5, wherein the operation treatment instrument (61) includes the wire cutter (62), and part of the wire cutter (62) is adapted to be capable of passing through the slit (48a) from inside the lumen (44) to protrude from the slit (48a), so that a distal end part of the operation treatment instrument (61) is formed into the arcuate shape.

7. The system including the elongated medical member (40) and the operation treatment instrument (61) according to any of claims 1 to 6, further comprising the insertion portion (42) having a long length, which is provided with a plurality of lumens through which the operation treatment instrument (61) can be penetrated.

8. The system including the elongated medical member (40) and the operation treatment instrument (61) according to claim 1, wherein the proximal end area (42) has a substantially circular cross sectional shape with a predetermined outer diameter.

9. The system including the elongated medical member (40) and the operation treatment instrument (61) according to claim 1, further comprising a holding portion (46, 56) provided to the guide portion (41) and adapted for holding the operation treatment instrument (59) extended along the guide portion (41) such that the operation treatment instrument (59) is prevented from moving axially away from the guide portion (41) at least a distal end of the guide portion (41).

## Patentansprüche

1. System, umfassend eine längliche medizinische Einheit (40) und ein Operationsbehandlungsinstrument (61), das System umfassend:
einen eine lange Länge aufweisenden Einführungsabschnitt (42), der mit einem Lumen (44) bereitgestellt ist, durch welchen ein Operationsbehandlungsinstrument (61) eingeführt werden kann,
wobei das Operationsbehandlungsinstrument (61) dazu eingerichtet ist, in eine bogenförmige Form durch ein distales Ende davon geformt zu werden, das angezogen wird, und eine Drahtschere (62) zum Schneiten eines lebenden Körpergewebes beinhaltet, wobei das Lumen (44) entlang einer longitudinalen Achse des Einführungsabschnitts (42) bereitgestellt ist,
wobei
der Einführungsabschnitt (42) biegbar ist und eine distale Endfläche beinhaltet, die einen Öffnungsabschnitt (44a) des Lumens (44) bei einer distalen Endfläche des Einführungsabschnitts (42) und eine proximale Endfläche beinhaltet, die eine vorbestimmte Länge zu einer proximalen Endfläche in einer Axialrichtung von der distalen Endfläche aufweist;
die längliche medizinische Einheit weiter einen Führungsabschnitt (41) umfasst, der zum Führen des sich von dem Lumen (44) erstreckenden Operationsbehandlungsinstruments (61) in eine Körperhöhle entlang des Führungsabschnitts (41) zusammen mit dem Einführungsabschnitt (42) eingerichtet ist, wobei ein proximales Ende zu einem peripheren Abschnitt des Öffnungsabschnitts (44a) des Lumens (44) verbunden ist und der Führungsabschnitt (41) bereitgestellt ist, um sich von dem proximalen Ende zu einem distalen Ende hin zu einer Richtung zu erstrecken, in welcher der Öffnungsabschnitt (44a) geöffnet ist, um einen distalen Endteil des Operationsbehandlungsinstruments (61), der von dem Öffnungsabschnitt (44a) des Lumens (44) hervorsteht, zu einem Zielbereich zu führen, und
einen Spalt (48a), der eine vorbestimmte Länge aufweist und von einem distalen Ende eines peripheren Abschnitts des Öffnungsabschnitts (44a) in einer proximalsten Fläche des Öffnungsabschnitts (44a) gebildet ist, wobei der Spalt (44a) bei einem proximalen Endteil davon ein langes Loch (48b) beinhaltet und dazu eingerichtet ist, es der Drahtschere (62) zu erlauben, von einem Inneren des Lumens (44), ohne dass eine Gesamtheit der Drahtschere (62) aus dem Öffnungsabschnitt (44a) herausgezogen wird, hindurchzureichen.

2. System, beinhaltend die längliche medizinische Einheit (40) und das Operationsbehandlungsinstrument (61) nach Anspruch 1, wobei der Führungsabschnitt (41) elastisch verformt werden kann.

3. System, beinhaltend die längliche medizinische Einheit (40) und das Operationsbehandlungsinstrument (61) nach Anspruch 1 oder 2, wobei eine Öffnung (45a) auf einer distalen Endfläche des Führungsabschnitts (41) gebildet ist, und ein Lumen zum Versrogen mit Flüssigkeit (45) in dem Einführungsabschnitt (42) und dem Führungsabschnitt (41) gebildet ist.

4. System, beinhaltend die längliche medizinische Einheit (40) und das Operationsbehandlungsinstrument (61) nach einem der Ansprüche 1 bis 3, wobei bei wenigstens einem Lochabschnitt (47a, 47b), welcher in Kommunikation mit dem Lumen (44) ist, an einem distalen peripheren Endseiten-Abschnitt des Einführungsabschnitts (42) gebildet ist.

5. System, beinhaltend die längliche medizinische Einheit (40) und das Operationsbehandlungsinstrument (61) nach einem der Ansprüche 1 bis 4, wobei ein Nutabschnitt (56), der in Kommunikation mit dem Lumen (44) des Einführungsabschnitts (42) ist und sich zu dem Führungsabschnitt (41) erstreckt, gebildet ist, wobei der Nutabschnitt (56) eine Form aufweist, die im Wesentlichen mit einem Eingriffsabschnitt (58) übereinstimmt, welcher zu dem Operationsbehandlungsinstrument bereitgestellt ist.

6. System, beinhaltend die längliche medizinische Einheit (40) und das Operationsbehandlungsinstrument (61) nach einem der Ansprüche 1 bis 5, wobei das Operationsbehandlungsinstrument (61) die Drahtschere (62) beinhaltet, und ein Teil der Drahtschere (62) dazu eingerichtet ist, dazu in der Lage zu sein, den Spalt (48a) aus einem Inneren des Lumens (44) zu durchlaufen, um von dem Spalt (48a) hervorzustehen, sodass ein distaler Endteil des Operationsbehandlungsinstruments (61) in der bogenförmigen Form gebildet ist.

7. System, beinhaltend die längliche medizinische Einheit (40) und das Operationsbehandlungsinstrument (61) nach einem der Ansprüche 1 bis 6, weiter umfassend, dass der Einführabschnitt (42) eine lange Länge aufweist, welche mit einer Vielzahl von Lumen bereitgestellt ist, durch welche das Operationsbehandlungsinstrument (61) hindurchgesteckt werden kann.

8. System, beinhaltend die längliche medizinische Einheit (40) und das Operationsbehandlungsinstrument (61) nach Anspruch 1, wobei die proximale Endfläche (42) eine im Wesentlichen kreisförmige Querschnittsform mit einem vorbestimmten Außendurchmesser aufweist.

9. System, beinhaltend die längliche medizinische Einheit (40) und das Operationsbehandlungsinstrument (61) nach Anspruch 1, weiter umfassend einen Halteabschnitt (46, 56), der zu dem Führungsabschnitt (41) bereitgestellt und zum Halten des Operationsbehandlungsinstruments (59) eingerichtet ist, das entlang des Führungsabschnitts (41) verlängert ist, sodass das Operationsbehandlungsinstrument (59) davor geschützt ist, sich axial weg von dem Führungsabschnitt (41) wenigstens einem distalen Ende des Führungsabschnitts (41) zu bewegen.

## Revendications

1. Système comprenant un élément médical allongé (40) et un instrument de traitement opérationnel (61), le système comprenant :
une partie d'insertion (42) ayant une longueur longue, qui est prévue avec une lumière (44) à travers laquelle un instrument de traitement opérationnel (61) peut être fait pénétrer, l'instrument de traitement opérationnel (61) étant adapté à être formé en une forme en arc par une extrémité distale de celui-ci étant tirée, et incluant un fil coupant (62) pour couper un tissu de corps vivant, la lumière (44) étant prévue le long d'un axe longitudinal de la partie d'insertion (42),
dans lequel
la partie d'insertion (42) peut être courbée et inclut une zone d'extrémité distale incluant une partie d'ouverture (44a) de la lumière (44) au niveau d'une surface d'extrémité distale de la partie d'insertion (42) et une zone d'extrémité proximale ayant une longueur prédéterminée vers un côté d'extrémité proximale dans un sens d'axe depuis la zone d'extrémité distale ;
l'élément médical allongé comprend en outre une partie de guidage (41) adaptée à guider l'instrument de traitement opérationnel (61) étendu depuis la lumière (44) jusque dans une cavité corporelle le long de la partie de guidage (41) ensemble avec la partie d'insertion (42), dans lequel une extrémité proximale est connectée à une partie périphérique de la partie d'ouverture (44a) de la lumière (44) et la partie de guidage (41) est prévue pour s'étendre de l'extrémité proximale jusqu'à une extrémité distale vers une direction dans laquelle la partie d'ouverture (44a) est ouverte, afin de guider une partie d'extrémité distale de l'instrument de traitement opérationnel (61) saillant depuis la partie d'ouverture (44a) de la lumière (44) jusqu'à une zone cible, et
une fente (48a) ayant une longueur prédéterminée et formée depuis une extrémité distale d'une partie périphérique de la partie d'ouverture (44a) dans une zone la plus proximale de la partie d'ouverture (44a), la fente (48a) incluant au niveau d'une partie d'extrémité proximale de celle-ci un trou long (48b) et étant adaptée à permettre que le fil coupant (62) passe à travers depuis l'intérieur de la lumière (44) sans qu'une totalité du fil coupant (62) ne soit guidée hors de la partie d'ouverture (44a).

2. Système incluant l'élément médical allongé (40) et l'instrument de traitement opérationnel (61) selon la revendication 1, dans lequel la partie de guidage (41) peut être élastiquement déformée.

3. Système incluant l'élément médical allongé (40) et l'instrument de traitement opérationnel (61) selon la revendication 1 ou 2, dans lequel une ouverture (45a) est formée sur une surface d'extrémité distale de la partie de guidage (41), et une lumière (45) d'amenée de fluide est formée dans la partie d'insertion (42) et la partie de guidage (41).

4. Système incluant l'élément médical allongé (40) et l'instrument de traitement opérationnel (61) selon l'une quelconque des revendications 1 à 3, dans lequel au moins une partie de trou (47a, 47b) qui est en communication avec la lumière (44) est formée sur une partie périphérique extérieure de côté d'extrémité distale de la partie d'insertion (42).

5. Système incluant l'élément médical allongé (40) et l'instrument de traitement opérationnel (61) selon l'une quelconque des revendications 1 à 4, dans lequel une partie de rainure (56) qui est en communication avec la lumière (44) de la partie d'insertion (42) et est étendue jusqu'à la partie de guidage (41) est formée, la partie de rainure (56) ayant une forme sensiblement coïncidente avec une partie d'engagement (58) prévue sur l'instrument de traitement opérationnel.

6. Système incluant l'élément médical allongé (40) et l'instrument de traitement opérationnel (61) selon l'une quelconque des revendications 1 à 5, dans lequel l'instrument de traitement opérationnel (61) inclut le fil coupant (62), et une partie du fil coupant (62) est adaptée à être apte à passer à travers la fente (48a) depuis l'intérieur de la lumière (44) pour faire saillie depuis la fente (48a), de telle manière qu'une partie d'extrémité distale de l'instrument de traitement opérationnel (61) est formée en une forme en arc.

7. Système incluant l'élément médical allongé (40) et l'instrument de traitement opérationnel (61) selon l'une quelconque des revendications 1 à 6, comprenant en outre la partie d'insertion (42) ayant une longueur longue, qui est prévue avec une pluralité de lumières à travers lesquelles l'instrument de traitement opérationnel (61) peut être fait pénétrer.

8. Système incluant l'élément médical allongé (40) et l'instrument de traitement opérationnel (61) selon la revendication 1, dans lequel la zone d'extrémité proximale (42) a une forme en coupe transversale sensiblement circulaire avec un diamètre extérieur prédéterminé.

9. Système incluant l'élément médical allongé (40) et l'instrument de traitement opérationnel (61) selon la revendication 1, comprenant en outre une partie de maintien (46, 56) prévue sur la partie de guidage (41) et adaptée à maintenir l'instrument de traitement opérationnel (59) étendu le long de la partie de guidage (41) de telle manière que l'instrument de traitement opérationnel (59) est empêché de déplacer axialement à l'écart de la partie de guidage (41) au moins une extrémité distale de la partie de guidage (41).
